(19) Europäisches Patentamt
European Patent Office
Office européen des brevets

(11) Publication number: 0 000 473
A1

(12) # EUROPEAN PATENT APPLICATION

(21) Application number: 78100207.6

(22) Date of filing: 21.06.78

(51) Int. Cl.$^2$: C 07 H 15/22, A 61 K 31/70

(30) Priority: 24.06.77 US 809839

(43) Date of publication of application: 07.02.79 Bulletin 79/3

(84) Designated contracting states: BE CH DE FR GB NL SE

(71) Applicant: SCHERICO LTD.
Töpferstrasse 5
CH-6004 Lucerne. (CH)

(72) Inventor: Daniels, Peter
11 Rockledge Place Cedar Grove
New Jersey 07009. (US)

(72) Inventor: McCombie, Stuart
159 Pleasant Valley Way West Orange
New Jersey 07052. (US)

(72) Inventor: Tattanahalli, L. Nagabhushan
3 Sunset Lane Parsippany
New Jersey 07054. (US)

(72) Inventor: Weinstein, Jay
40 Conger Street Bloomfield
New Jersey 07003. (US)

(74) Representative: Antony, Fritz, Dr. et al
P.O. Box 601 Winkelriedstrasse 35
CH-6002 Lucerne. (CH)

(54) **Process for preparing aminoglycoside derivatives, novel derivatives obtained and pharmaceutical compositions containing such derivatives.**

(57) This invention relates to a process for preparing 2'-hydroxy- 2'-desamino-4,6-di-*O*-(aminoglycosyl) -1,3-diaminocyclitols having a 6'-amino function and pharmaceutically acceptable acid addition salts thereof, to novel compounds and salts obtained by this process; and to novel antibacterially active compositions comprising such novel compounds or salts.

The process comprises reacting the corresponding *N*-protected (except the 2'-amino)-4,6- di-*O*- (aminoglycosyl)-1,3-diaminocyclitol with hydrogen peroxide in the presence of tungstate ion, followed by cleavage of the thereby formed 2'-oximino derivative and by reduction of the resulting 2'-oxo derivative and by removal of the *N*-protecting groups.

The preferred group of novel compounds obtained by this process are the 1-*N*- (ω-amino-α- hydroxyalkanoyl) -2'-hydroxy-2'- desamino-4,6-di-*O*- (aminoglycosyl)- 1,3-diaminocyclitols. The compounds obtained by the process of this invention exhibit antibacterial activity

EP 0 000 473 A1

# PROCESS FOR PREPARING AMINOGLYCOSIDE DERIVATIVES, NOVEL DERIVATIVES OBTAINED AND PHARMACEUTICAL COMPOSITIONS CONTAINING SUCH DERIVATIVES

This invention relates to a process for preparing 2'-hydroxy-2'-desamino-4,6-di-O-(aminoglycosyl)-1,3-diaminocyclitols having a 6'-amino function and pharmaceutically acceptable acid addition salts thereof, to novel compounds and salts obtained by this process; and to novel antibacterially active compositions comprising such novel compounds or salts.

4,6-Di-O-(aminoglycosyl)-1,3-diaminocyclitols are generally known in the art. Some of these, e.g. kanamycin A, gentamicin B and gentamicin $B_1$ have a 2'-hydroxy-group and a 6'-amino function. They are, however, obtained microbiologically, e.g. gentamicin B and $B_1$ are coproduced with the gentamicin C complex during the fermentation of certain species of the genus *Micromonospora*, e.g. M. purpurea as described in U.S. Patents No. 3,091,572 and 3,915,955; and kanamycin is produced microbiologically by the fermentation of certain species of the genus *Streptomyces*, e.g. S. kanamyceticus as described in U.S. Patent No. 2,931,798.

The process of this invention provides a chemical method for preparing novel 2'-hydroxy-2'-desamino-4,6-di-O-(aminoglycosyl)-1,3-diaminocyclitols having antibacterial activity which cannot be obtained via prior art microbiological methods.

In addition the process provides a novel method for preparing known compounds, e.g. gentamicin B (which is obtained in poor yields, i.e. less than about 10 %, when prepared microbiologically) by converting the known Antibiotic JI-20A into 2'-hydroxy-2'-desamino-Antibiotic JI-20A which has the same structure as gentamicin B.

Typical representatives of the novel compounds obtained by the process of this invention are

2'-hydroxy-2'-desaminogentamicin $C_1$,
2'-hydroxy-2'-desaminogentamicin $C_{1a}$,
2'-hydroxy-2'-desaminogentamicin $C_2$,
2'-hydroxy-2'-desaminogentamicin $C_{2a}$,
2'-hydroxy-2'-desaminogentamicin $C_{2b}$,
2'-hydroxy-2'-desaminosisomicin,
2'-hydroxy-2'-desaminoverdamicin,
2'-hydroxy-2'-desamino-Antibiotic G-52,
2'-hydroxy-2'-desamino-Antibiotic 66-40B,
2'-hydroxy-2'-desamino-Antibiotic 66-40D,
2'-hydroxy-2'-desamino-Antibiotic JI-20B,
2'-hydroxy-2'-desaminotobramycin; and
2'-hydroxy-2'-desamino-3',4'-dideoxykanamycin B;
the 5-deoxy-, 5-epi-, 5-epi-azido-5-deoxy- and the 5-epi-fluoro-5-deoxy derivatives thereof;
2'-hydroxy-2'-desamino-Antibiotic MU-1, and
2'-hydroxy-2'-desamino-Antibiotic Mu-4;

and the 1-<u>N</u>-mono-substituted derivatives thereof wherein the substituent R is either an (ω-amino-α-hydroxy-alkanoyl) group having from 3 to 5 carbon atoms or a group $CH_2X$ wherein X represents hydrogen, alkyl, alkenyl, cycloalkyl, cycloalkylalkyl, aryl, aralkyl, hydroxyalkyl, aminoalkyl, N-alkylaminoalkyl, aminohydroxyalkyl or alkylaminohydroxyalkyl, said aliphatic radicals having up to 7 carbon atoms

and, when substituted by both hydroxy and amino, having said substituents attached to different carbon atoms; and the pharmaceutically acceptable acid addition salts thereof.

The antibiotics gentamicin $C_1$, $C_{1a}$, $C_2$, $C_{2a}$, $C_{2b}$, sisomicin, verdamicin, Antibiotic G-52, Antibiotic 66-40B, Antibiotic 66-40D, Antibiotic JI-20B, tobramycin, 3',4'-dideoxykanamycin B, Antibiotic Mu-1 and Antibiotic Mu-4, as well as Antibiotic JI-20A (which may be converted into gentamicin B by the process of this invention) may be illustrated by the following formula I

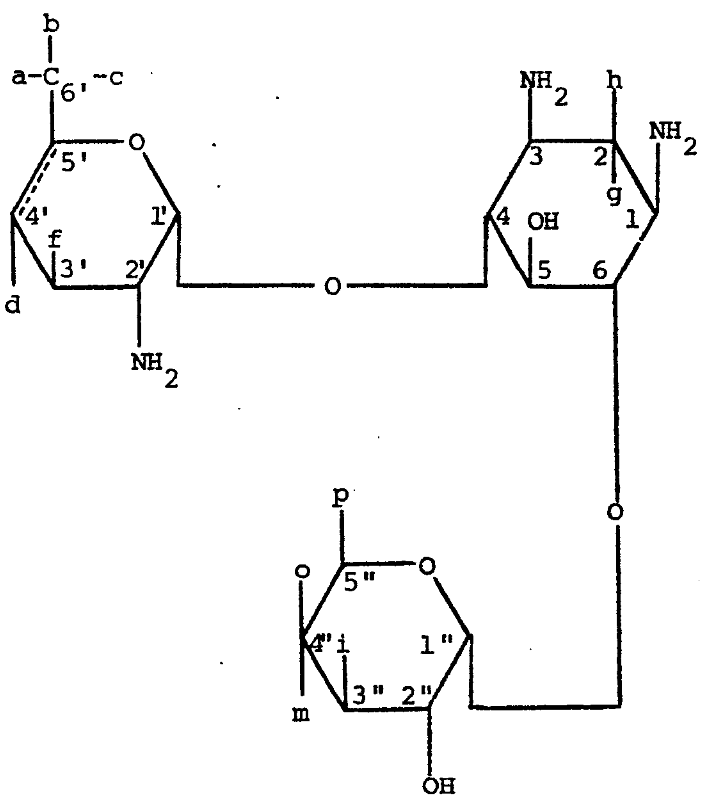

I

whereby, for the various antibiotics listed above, the "substituents" a, b, c, d, f, g, h, i, m, o and p as well as the bond between C4' and C5' are as given in the table below.

TABLE I

| | | a (6') | b (6') | c (6') | d (4') | f (3') | g (2) | h (2) | i (3") | m (4") | o (4") | p (5") | Bond 4'-5' |
|---|---|---|---|---|---|---|---|---|---|---|---|---|---|
| 1 | Gentamicin $C_1$ | $CH_3NH$ | $CH_3$ | H | H | H | H | H | $NHCH_3$ | $CH_3$ | OH | H | single |
| 2 | Gentamicin $C_{1a}$ | H | H | $NH_2$ | H | H | H | H | $NHCH_3$ | $CH_3$ | OH | H | single |
| 3 | Gentamicin $C_2$ | $NH_2$ | $CH_3$ | H | H | H | H | H | $NHCH_3$ | $CH_3$ | OH | H | single |
| 4 | Gentamicin $C_{2a}$ | H | $CH_3$ | $NH_2$ | H | H | H | H | $NHCH_3$ | $CH_3$ | OH | H | single |
| 5 | Gentamicin $C_{2b}$ | H | H | $NHCH_3$ | H | H | H | H | NHCH | $CH_3$ | OH | H | single |
| 6 | Sisomicin | H | H | $NH_2$ | H | H | H | H | $NHCH_3$ | $CH_3$ | OH | H | double |
| 7 | Verdamicin | H | $CH_3$ | $NH_2$ | H | H | H | H | $NHCH_3$ | $CH_3$ | OH | H | double |
| 8 | Antibiotic G-52 | H | H | $NHCH_3$ | H | H | H | H | $NHCH_3$ | $CH_3$ | OH | H | double |
| 9 | Antibiotic 66-40B | H | H | $NH_2$ | H | H | H | H | $NHCH_3$ | OH | H | H | double |
| 10 | Antibiotic 66-40D | H | H | $NH_2$ | H | H | H | H | $NHCH_3$ | H | OH | H | double |
| 11 | Antibiotic JI-20B | H | $CH_3$ | $NH_2$ | OH | OH | H | H | $NHCH_3$ | $CH_3$ | OH | H | single |
| 12 | Tobramycin | H | H | $NH_2$ | OH | H | H | H | $NH_2$ | OH | H | $CH_2OH$ | single |
| 13 | 3',4'-dideoxykanamycin B | H | H | $NH_2$ | H | H | H | H | $NH_2$ | OH | H | $CH_2OH$ | single |
| 14 | Antibiotic Mu-1 | H | H | $NH_2$ | H | H | OH | H | $NHCH_3$ | $CH_3$ | OH | H | double |
| 15 | Antibiotic Mu-4 | H | H | $NH_2$ | H | H | H | OH | $NHCH_3$ | $CH_3$ | OH | H | double |
| 16 | Antibiotic JI-20A | H | H | $NH_2$ | OH | OH | H | H | $NHCH_3$ | $CH_3$ | OH | H | single |

Included among the substituents contemplated for the grouping $CH_2X$ referred to above are straight and branched chain alkyl groups such as ethyl, n-propyl, n-butyl, β-methylpropyl; cycloalkyl groups such as cyclopropylmethyl; alkenyl groups such as β-propenyl, β-methylpropenyl, β-butenyl; hydroxy substituted straight and branched chain alkyl groups such as β-hydroxy-γ-methylbutyl, β-hydroxy-β-methylpropyl, α-hydroxybutyl, β-hydroxypropyl, γ-hydroxypropyl; amino substituted straight and branched chain alkyl groups such as β-aminopropyl, γ-aminopropyl, α-aminobutyl, and mono-N-alkylated derivatives thereof such as the N-methyl and N-ethyl derivatives, e.g. β-methylaminopropyl; amino and hydroxy disubstituted straight and branched chain alkyl groups such as β-hydroxy-δ-aminobutyl, β-hydroxy-γ-aminopropyl, and β-hydroxy-β-methyl-γ-aminopropyl; and mono-N-alkylated derivatives thereof such as β-hydroxy-γ-ethylaminopropyl.

Of the foregoing substituents for the moiety $CH_2X$, preferred are ethyl, n-propyl and δ-aminobutyl.

Of the 1-N-substituted derivatives of our invention, preferred are the 1-N-(ω-amino-α-hydroxyalkanoyl)-2'-hydroxy-2'-desamino-4,6-di-O-(aminoglycosyl)-1,3-diaminocyclitols, particularly the 1-N-(γ-amino-α-hydroxybutyryl) and the 1-N-(β-amino-α-hydroxypropionyl) derivatives, particularly valuable compounds being 1-N-(S-γ-amino-α-hydroxybutyryl)-2'-hydroxy-2'-desaminogentamicin $C_{1a}$ and 1-N-(S-β-amino-α-hydroxypropionyl)-2'-hydroxy-2'-desaminogentamicin $C_{1a}$.

It is to be noted that the 1-N-(ω-amino-α-hydroxy) substituent in the foregoing 1-N-(ω-amino-α-hydroxy)-2'-hydroxy-2'-desamino-4,6-di-O-(aminoglycosyl)-1,3-diaminocyclitols may be in the R,S- form or in the R- form or in the S- form. In accordance with this invention, each of the foregoing names

includes all three forms. Thus, the name 1-N-(γ-amino-α-hydroxybutyryl)-2'-hydroxy-2'-desaminogentamicin $C_{1a}$ includes 1-N-(S-γ-amino-α-hydroxybutyryl)-2'-hydroxy-2'-desaminogentamicin $C_{1a}$, 1-N-(R-γ-amino-α-hydroxybutyryl)-2'-hydroxy-2'-desaminogentamicin $C_{1a}$ and 1-N-(R,S-γ-amino-α-hydroxybutyryl)-2'-hydroxy-2'-desaminogentamicin $C_{1a}$.

The pharmaceutically acceptable acid addition salts of this invention may be prepared according to known procedures such as by neutralizing the free base with the appropriate acid, usually to about pH 5. Preferred acids for this purpose are hydrochloric, sulfuric, phosphoric and hydrobromic acid. Generally, the acid addition salts are white solids which are soluble in water and insoluble in most polar and nonpolar organic solvents.

The process of this invention for the preparation of a 2'-hydroxy-2'-desamino-4,6-di-O-(aminoglycosyl)-1,3-diaminocyclitol having an amino function in position 6' is characterised in that the corresponding 4,6-di-O-(aminoglycosyl)-1,3-diaminocyclitol having an amino function in position 2' wherein all other amino functions are protected by a protecting group, is oxidized by means of hydrogen peroxide in an inert solvent in the presence of tungstate ion at a pH of about 9 to about 11, whereby the molar ratio of hydrogen peroxide to starting compound is at least 2:1, that the so-obtained 2'-oximino-4,6-di-O-(aminoglycosyl)-1,3-diaminocyclitol is subjected to a cleavage of the oxime function; that the so-obtained 2'-oxo-4,6-di-O-(aminoglycosyl)-1,3-diaminocyclitol is reduced at the oxo group; and that the so-obtained compound is subjected to a removal of the protecting groups.

For the oxidation in the first step of the process it is necessary to use at least two moles of hydrogen peroxide per

mole of aminoglycoside to ensure completion of reaction. Preferably, a four to five molar excess is used, and in certain instances even greater molar excesses of hydrogen peroxide are advantageously employed.

The oxidation is usually carried out at room temperature in aqueous methanol, although other lower alkanols, e.g. ethanol and isopropanol, are conveniently employed. In general, however, any organic solvent which will dissolve the aminoglycoside and which does not react with hydrogen peroxide, e.g. dimethylacetamide, dimethylformamide, tetrahydrofuran, dioxan and diglyme may be used.

After addition of the tungstate ion, it is necessary that the pH of the reaction mixture be maintained between 9 and 11 (preferably at pH 10 to pH 10.5), e.g. by addition of a base (sodium hydroxide), in order to minimize side reactions occurring such as glycoside cleavage and protonation of the amine.

The reaction is continued until almost all the amino function is consumed as determined by thin layer chromatographic techniques, then the resulting 2'-oximino-N-protected-4,6-di-O-(aminoglycosyl)-1,3-diaminocyclitol intermediate is isolated as a mixture of syn and anti isomers utilizing known techniques (e.g. extraction and chromatographic techniques).

In the second step of the process, the 2'-oximino-N-protected -4,6-di-O-(aminoglycosyl)-1,3-diaminocyclitol prepared as described above is cleaved to the corresponding 2'-oxo derivative utilizing known techniques for cleaving oximes. Preferably, the cleaving of the 2'-oximino function is achieved by an acidic hydrolytic medium in the presence of acetaldehyde (e.g. by treating a solution of the 2'-oximino derivative in acetonitrile with 1N hydrochloric acid containing acetaldehyde) or by reaction of the oximino intermediate with sodium bisulfite followed by mild acid hydrolysis. The latter procedure is preferred since greater yields of purer product are usually obtained thereby. When carrying out this procedure, the 2'-oximino-N-protected aminoglycoside intermediate in aqueous ethanol (or other aqueous lower alkanol) is usually reacted with about three to four molar equivalents of sodium bisulfite at reflux temperature until the oximino function is consumed as determined by thin layer chromatographic techniques. There is then added a weak acid (e.g. acetic acid or oxalic or tartaric acid) to produce a mild acid hydrolytic medium; the reaction mixture is then heated at reflux temperature until the reaction is complete as evidenced by thin layer chromatographic techniques.

Reduction of the 2'-oxo-N-protected aminoglycoside intermediate in the third step is preferentially carried out by reaction with an alkali metal borohydride (usually sodium borohydride) according to known procedures for reducing a keto group to a hydroxyl function, and there is obtained an excellent yield of N-protected-2'-hydroxy-2'-desamino-aminoglycoside in which the 2'-hydroxyl function is in the equatorial position, i.e. the same stereoconfiguration as the starting 2'-amino function. After removal of the N-protecting functions according to known techniques, the resulting 2'-hydroxy-2'-desamino-4,6-di-O-(aminoglycosyl)-1,3-diaminocyclitol is isolated and purified utilizing conventional techniques.

When carrying out the process, all the functions desired in the final 2'-hydroxy-2'-desamino derivative may be present in the 4,6-di-O-(aminoglycosyl)-1,3-diaminocyclitol precursor. Thus, for example, when preparing 1-N-ethyl-2'-hydroxy-

2'-desaminosisomicin, or 5-deoxy-2'-hydroxy-2'-desaminosisomicin, the corresponding 1-N-ethylsisomicin or 5-deoxysisomicin may be used as starting compounds. Alternatively, the 2'-amino function in a 4,6-di-O-(aminoglycosyl)-1,3-diaminocyclitol can be replaced by a hydroxyl group via the process of this invention, then other functions may be introduced into the 2'-hydroxy-2'-desamino-aminoglycoside by methods known in the art. This alternate method is the method of choice when preparing the preferred 1-N-(ω-amino-α-hydroxyalkanoyl)-2'-hydroxy-2'-desamino derivatives of this invention. Thus, for example, when preparing 1-N-(S-β-amino-α-hydroxy-propionyl)-2'-hydroxy-2'-desaminogentamicin $C_{1a}$ and 1-N-(γ-amino-α-hydroxybutyryl)-2'-hydroxy-2'-desaminogentamicin $C_{1a}$, a preferred method involves first converting gentamicin $C_{1a}$ to 2'-hydroxy-2'-desaminogentamicin $C_{1a}$ via the process of this invention, and thence converting 2'-hydroxy-2'-desaminogentamicin $C_{1a}$ to the 1-N-(S-β-amino-α-hydroxypropionyl)- or the 1-N-(S-γ-amino-α-hydroxybutyryl)-2'-hydroxy-2'-desaminogentamicin $C_{1a}$ utilizing techniques known in the art and as described in the examples.

Typical starting compounds are the 4,6-di-O-(aminoglycosyl)-1,3-diaminocyclitol antibacterial agents described by formula I in combination with Table I above. Particularly useful starting antibiotic precursors are sisomicin and gentamicin $C_{1a}$ which lead to preferred 2'-hydroxy-2'-amino derivatives of this invention, and Antibiotic JI-20A which leads to gentamicin B.

Other useful starting antibiotic precursors include 5-epi-4,6-di-O-(aminoglycosyl)-1,3-diaminocyclitol antibacterials described in U.S. Patent No. 4,000,261; 5-epi-azido-5-deoxy-4,6-di-O-(aminoglycosyl)-1,3-diaminocyclitol antibacterials described in U.S. Patent No. 4,000,262; 1-N-alkyl-4,6-di-O-(aminoglycosyl)-1,3-diaminocyclitol antibacterials described in U.S. Patent No. 4,002,742; 5-deoxy-4,6-di-O-(aminoglycosyl)-1,3-diaminocyclitol antibacterials described in U.S. Patent No. 4,053,591; and 5-epi-fluoro-5-deoxy-4,6-di-O-(aminoglycosyl)-1,3-diaminocyclitol antibacterials described in South African Patent No. 78/0385.

A 2'-hydroxy-2'-desamino-4,6-di-O-(aminoglycosyl)-1,3-diaminocyclitol of this invention may be converted to a corresponding 1-N-alkyl derivative or to a corresponding 5-deoxy-, or a 5-epi- or 5-epi-azido-5-deoxy derivative, via procedures analogous to those described in the above-mentioned patents.

Thus, for example, a 2'-hydroxy-2'-desamino-4,6-di-O-(aminoglycosyl)-2-deoxystreptamine of this invention (e.g. 2'-hydroxy-2'-desaminosisomicin) is converted to the corresponding 5-deoxy derivative (e.g. 2'-hydroxy-2'-desamino-5-deoxysisomicin) by first converting said 2'-hydroxy-2'-desamino-4,6-di-O-(aminoglycosyl)-2-deoxystreptamine to the corresponding N- and O-protected (except for the 5-hydroxyl group) intermediate (e.g. 1,3,6'-tri-N-benzyloxycarbonyl-2',2"-di-O-benzoyl-3",4"-N,O-carbonyl-2'-hydroxy-2'-desaminosisomicin) utilizing known techniques, then converting the 5-hydroxyl group to the 5-O-thioformate ester by reaction with the Vilsmaier salt produced from phosgene and N,N-dimethylformamide in dichloromethane, followed by reaction of the imidinium chloride salt thereby produced (e.g. 1,3,6'-tri-N-benzyloxycarbonyl-2',2"-di-O-benzoyl-3",4"-N,O-carbonyl-2'-hydroxy-2'-desaminosisomicin-5-O-(N,N-dimethylformimidinium)chloride) with hydrogen sulfide in pyridine, thence reaction of the requisite 5-O-thioformyl ester thereby formed (e.g. 1,3,6'-tri-N-benzyloxycarbonyl-5-O-thioformyl-2',2"-di-O-benzoyl-3",4"-N,O-carbonyl-2'-hydroxy-2'-desaminosisomicin) with tri-n-butylstannane in toluene and, finally, removal of the N- and O-protecting groups in the resulting 5-deoxy derivative via known procedures to obtain a 5-deoxy-2'-desamino-2'-hydroxy compound of this invention (e.g. 2'-hydroxy-2'-desamino-5-deoxysisomicin).

Similarly, a 2'-hydroxy-2'-desamino-4,6-di-O-(aminoglycosyl)-2-deoxystreptamine of this invention is converted to the corresponding 5-epi-fluoro-5-deoxy derivative according to procedures analogous to those described in South African Patent No. 78/0385, whereby an N- and O-protected-2'-hydroxy-2'-desamino-4,6-di-O-(aminoglycosyl)-2-deoxystreptamine having a free 5-hydroxyl group (e.g. 1,3,6'-tri-N-benzyloxycarbonyl-2',2"-di-Obenzoyl-3",4"-N,O-carbonyl-2'-hydroxy-2'-desaminosisomicin) is reacted with a dialkylaminosulfur trifluoride, preferably diethylaminosulfur trifluoride in methylene chloride, followed by treatment with sodium bicarbonate and then removal of the N- and O-protecting groups to obtain a 5-epi-fluoro-2'-hydroxy-2'-desamino-4,6-di-O-(aminoglycosyl)-2,5-dideoxystreptamine of this invention (e.g. 5-epi-fluoro-2'-hydroxy-2'-desamino-5-deoxysisomicin).

The N-protected starting compounds of our process, wherein all amino functions except the 2'-amino group in a 4,6-di-O-(aminoglycosyl)-1,3-diaminocyclitol are protected by groups susceptible to reductive cleavage or basic hydrolysis, are conveniently prepared by combining procedures known in the art, together with a selective blocking procedure described in Belgian Patent No. 855704. By the selective blocking procedure, a transition metal salt complex between available neighboring amino and hydroxyl functions in said 4,6-di-O-(aminoglycosyl)-1,3-diaminocyclitol and cupric acetate or nickel (II) acetate is first prepared followed by introduction of acyl functions on non-complexed and/or

weakly complexed amino functions, and thence removal of said transition metal salt complex by means of hydrogen sulfide or ammonium hydroxide.

When preparing N-protected starting compounds of saturated aminoglycosides having a primary carbinamine at C-5', e.g. 1,3,6',3"-tetra-N-protected gentamicin $C_{1a}$, it is usually preferred to block the amino functions by hydrocarbonyloxycarbonyl groups which are susceptible to reductive cleavage (e.g. with sodium in ammonia) or to alkaline hydrolysis, e.g. by utilizing blocking groups such as benzyloxycarbonyl, ethoxycarbonyl, and methoxycarbonyl groups, Such an N-protected intermediate, e.g. 1,3,6',3"-tetra-N-benzyloxycarbonylgentamicin $C_{1a}$, is conveniently prepared by first reacting the unblocked aminoglycoside, e.g. gentamicin $C_{1a}$, with about two equivalents of N-(trichloroethoxycarbonyloxy)succinimide in methanol in the presence of excess nickel acetate according to the selective blocking procedure whereby, after decomposition of the nickel acetate complex by means of ammonium hydroxide, there is produced 2',6'-di-N-trichloroethoxycarbonylgentamicin $C_{1a}$; secondly, preparing the N-benzyloxycarbonyl derivatives of the remaining amino functions at the 1, 3 and 3"-positions via the well known procedure utilizing benzylchloroformate and calcium hydroxide; thirdly, treating the resulting 1,3,3"-tri-N-benzyloxycarbonyl-2',6'-di-N-(trichloroethoxycarbonyl)-gentamicin $C_{1a}$ with zinc in acetic acid whereby are removed the trichloroethoxycarbonyl groups to produce 1,3,3"-tri-N-benzyloxycarbonylgentamicin $C_{1a}$; then, finally, blocking the more reactive 5'-primary carbinamine group by reaction of the 1,3,3"-tri-N-blocked derivative with about one equivalent of N-(benzyloxycarbonyloxy)phthalimide in the presence of excess triethylamine to produce the desired 1,3,6',3"-tetra-N-benzyloxycarbonylgentamicin $C_{1a}$, a requisite intermediate

of the process of this invention.

When preparing N-blocked aminoglycoside staring compounds from saturated 4,6-di-O-(aminoglycosyl)-1,3-diaminocyclitols having a secondary amino function at the 6'-position (such as in gentamicin $C_1$), the requisite 1,3,6',3"-tetra-N-blocked intermediate (e.g. 1,3,6',3"-tetra-N-ethoxycarbonylgentamicin $C_1$) is preferably prepared by first reacting gentamicin $C_1$ with about one equivalent of N-benzyloxycarbonyloxysuccinimide in the presence of about three equivalents of cupric acetate according to the selective blokking procedure whereby, after decomposition of the copper complex with ammonium hydroxide, there is obtained 2'-N-benzyloxycarbonylgentamicin $C_1$. Reaction of the foregoing derivative with over four equivalents of ethoxycarbonyl chloride and sodium carbonate, followed by removal of the 2'-benzyloxycarbonyl group via reductive cleavage by means of hydrogen in the presence of palladium-on-charcoal catalyst yields 1,3,6',3"-tetra-N-ethoxycarbonyl-gentamicin $C_1$.

When preparing 2'-hydroxy-2'-desamino-4,6-di-O-(aminoglycosyl)-1,3-diaminocyclitols having double bonds, such as in sisomicin, the N-blocking groups in the unsaturated aminocyclitol are preferably N-benzoyl blocking groups since such derivatives are less susceptible to glycoside cleavage under the acidic reaction conditions necessary for the deoximation step of this process. Thus, a preferred N-blocked sisomicin starting intermediate is 1,3,6'-tri-N-benzoyl-3"-N-acetylsisomicin, which is conveniently prepared by first treating sisomicin with excess N-benzoyl imidazole followed by treatment of the resulting 6'-N-benzoylsisomicin with one equivalent of N-(trichloroethoxycarbonyloxy)succinimide in methanol in the presence of nickel (II) acetate according to the selective blocking procedure whereby (after decomposition of the nickel acetate complex by means of ammonium hydroxide) is produced mainly 2'-trichloroethoxycarbonyl-6'-N-benzoylsisomicin. Treatment of the foregoing with excess benzoic anhydride in methanol yields 1,3,6'-tri-N-benzoyl-2'-(tri-chloroethoxycarbonyloxy)sisomicin which, upon reaction with excess acetic anhydride followed by treatment with zinc in acetic acid in methanol yields 1,3,6'-tri-N-benzoyl-3"-N-acetylsisomicin.

When preparing N-protected starting compounds from aminoglycosides having amino groups at the 2' and 6'-positions and hydroxyl groups at the 3' and 4'-positions (e.g. a 1,3,6'3"-tetra-N-protected-Antibiotic JI-20A), it is preferred to first protect the 6'-position by reacting the aminoglycoside with about one equivalent of N-benzyloxycar-bonyloxyphthalimide in the presence of about one equivalent of cupric chloride according to the selective blocking procedure whereby, after decomposition of the copper complex with ammonium hydroxide there is obtained the 6'-N-benzyloxycarbonyl derivative (e.g. 6'-N-benzyloxycarbonyl-Antibiotic JI-20A). The 2'-position is then protected by reaction of the foregoing 6'-N-substituted derivative with about a mole of either N-tert.-butoxycarbonyloxyphthalimide or N-(2,2,2-tri-chloroethoxy)carbonyloxyphthalimide in the presence of about an equivalent of nickel acetate according to the selective blocking method whereby, after decomposition of the nickel complex with ammonium hydroxide, the corresponding 2'-N-tert.-butoxycarbonyl or 2'-N-(2,2,2-trichloroethoxycarbonyl) derivative is formed (e.g. 2'-N-tert.-butoxycarbonyl- or 2'-N-(2,2,2-trichloroethoxycarbonyl)-6'-N-benzyloxycarbonyl-Anti-biotic JI-20A). Reaction of the foregoing 2',6'-di-N-protected aminoglycoside with over three equivalents of benzyloxy-carbonylchloride according to standard blocking

procedures, followed by removal fo the 2'-N-tert.-butoxycarbonyl-protecting group with trifluoroacetic acid or removal of the 2'-N-(2,2,2-trichloroethoxy)-protecting group by treatment with trifluoroacetic acid or zinc and acetic acid in methanol respectively yields a 1,3,6',3"-tetra-N-protected-Aminoglycoside starting compound of this invention, e.g. 1,3,6',3"-tetra-N-benzyloxy-Antibiotic JI-20A.

It will be obvious to one skilled in the art that other requisite 1,3,6',3"-tetra-N-acyl aminoglycoside starting compounds of this process can be prepared by other combinations of acylating procedures known in the art together with the selective blocking procedures disclosed in Belgian Patent No. 855704.

## PREPARATION I

## 1,3,6',3"-TETRA-N-BENZYLOXYCARBONYLGENTAMICIN $C_{1a}$

A. 2',6'-Di-N-(2,2,2-Trichloroethoxycarbonyl)Gentamicin $C_{1a}$

To a solution of gentamicin $C_{1a}$ (5.0 g) and nickel (II) acetate (10.0 g) in methanol (250 ml) at $0^{o}C$ with stirring add N-(2,2,2-trichloroethoxycarbonyloxy)succinimide (6.25 g). Allow the solution to reach room temperature and stir at room temperature for an hour, then evaporate *in vacuo* until the reaction solution has a volume of about 100 ml. Add 2 N ammonium hydroxide (500 ml), extract with chloroform (three 120 ml portions), dry the combined extracts with potassium carbonate and evaporate. Purify the resultant residue by chromatography on silica gel (300 g) eluting with a chloroform:methanol:concentrated ammonium hydroxide (4:1:0.1) solvent system. Combine the like eluates of desired product as determined by thin layer chromatography and evaporate. Dissolve the resultant residue in chloroform (30 ml), add the chloroform solution to a stirred mixture of hexane (400 ml)

and ether (100 ml). Separate the resultant precipitate by filtration and dry at 60$^{O}$C in vacuo (1 mm) to obtain 2',6'-di-N-(2,2,2-trichloroethoxycarbonyl)-gentamicin $C_{1a}$; yield 4.9 g; 62 % theory; m.p. 119-122$^{O}$C; $[\alpha]_D^{26}$ + 95.4$^{O}$ (chloroform, c=0.48).

B. 1,3,3"-Tri-N-Benzyloxycarbonylgentamicin $C_{1a}$

To a solution of 2,6'-di-N-(2,2,2-trichloroethoxycarbonyl)gentamicin $C_{1a}$ (17.0 g) and calcium hydroxide (25 g) in methanol (250 ml) cooled to 5$^{O}$C, add over a 2-minute interval benzoyl chloroformate (25 ml). Allow the solution to reach room temperature and stir at room temperature for 30 minutes. Dilute the reaction mixture (now containing 1,3,3"-tri-N-benzyloxycarbonyl-2',6-di-N-(2,2,2-trichloroethoxycarbonyl)gentamicin $C_{1a}$) with methanol (250 ml), water (100 ml) and acetic acid (75 ml). Add zinc dust (40 g) and stir at room temperature for 30 minutes, then stir and heat at reflux temperature for one hour. Cool, remove the zinc by filtration and wash with methanol (two portions of 25 ml). Add the combined filtrate and washings to a mixture of ice (1000 g) and concentrated ammonium hydroxide (500 ml). Extract the aqueous mixture with chloroform (800 ml), then two 300 ml portions. Wash the combined extracts with water and evaporate. Chromatograph the resultant residue on silica gel (350 g) eluting with a solvent mixture of chloroform:Methanol:concentrated ammonium hydroxide (10:1:0.1). Evaporate the combined, like eluates as determined by thin layer chromatography, dissolve the resultant residue in chloroform (50 ml), and add the chloroform solution dropwise to a stirred mixture of ether (100 ml) and hexane (700 ml). Separate the resultant precipitate by filtration and dry in vacuo at 60$^{O}$C to give 1,3,3"-tri-N-benzyloxycarbonylgentamicin $C_{1a}$;

yield 11.7 g; 65 % theory; m.p. 110-113$^{o}$C; $[\alpha]_D^{26}$ 96.5$^{o}$ (chloroform, c=0.55).

C. 1,3,6',3"-Tetra-N-Benzyloxycarbonylgentamicin $C_{1a}$

To a stirred solution of 1,3,3"-tri-N-benzyloxycarbonyl-gentamicin $C_{1a}$ (9.35 g) and triethylamine (6.0 ml) in chloroform (100 ml) at 0 - 5$^{o}$C, add dropwise over a 5-minute period a solution of N-(benzyloxycarbonyloxy) phthalimide (3.2 g) in chloroform (90 ml). Stir the reaction mixture an additional 10 minutes, then wash the reaction mixture with ammonium hydroxide (2 Normal). Dry over potassium carbonate and evaporate. Chromatograph the resultant residue on silica gel (200 g) eluting with a solvent mixture comprising chloroform:methanol:concentrated ammonium hydroxide (20:1:0.1). Combine the like eulates containing the desired product as determined by thin layer chromatography and evaporate. Dissolve the resultant residue in a minimum quantity of chloroform and add the chloroform dropwise to hexane (500 ml). Separate the resultant precipitate by filtration, dry *in vacuo* at 60$^{o}$C to give 1,3,6',3"-tetra-N-benzyloxycarbonylgentamicin $C_{1a}$; yield 8.5 g (79 % theory); m.p. 101 - 104$^{o}$C; $[\alpha]_D^{26}$ + 73.9$^{o}$ (chloroform, c=0.48).

## PREPARATION 2

### 3"-N-ACETYL-1,3,6'-TRI-N-BENZOYLSISOMICIN

A. 6'-N-Benzoylsisomicin

To a solution of sisomicin (10 g) in methanol (100 ml) at 0 - 5$^{o}$C, add dropwise a solution of N-benzoyl imidazole (7 g, 2 equivalents) in chloroform (30 ml). Stir the reaction mixture at room temperature for 30 minutes, concentrate *in vacuo* to a small volume, dissolve the resultant syrupy residue in choroform (70 ml) and add the chloroform solution slowly to stirred ether (650 ml).

Separate the resultant precipitate by filtration, wash with ether and dry in vacuo at 50°C to obtain 6'-N-benzoylsisomicin (14 g) which is used without further purification in the procedure of Preparation 2B.

B. 2'-N-(2,2,2-Trichloroethoxycarbonyl)-6'-N-Benzoylsisomicin

Dissolve 6'-N-benzoylsisomicin (14 g) in methanol (450 ml) and add nickel (II) acetate (25 g) and stir for 20 minutes at room temperature. Cool the reaction mixture to 0 - 5°C, and add dropwise a solution of N-(2,2,2-trichloroethoxycarbonyloxy)succinimide (6.4 g, 1.1 equivalents) in chloroform (40 ml). Stir at room temperature for 30 minutes, then add concentrated ammonium hydroxide (25 ml) and evaporate in vacuo to a volume of about 100 ml. Dilute the resultant residue with 3 N ammonium hydroxide (250 ml) and extract with chloroform (two 200 ml portions). Dry the combined extracts over potassium carbonate, filter and evaporate in vacuo to a residue comprising 2'-N-(2,2,2-trichloroethoxycarbonyl)-6'-N-benzoylsisomicin, which is used without further purification in the procedure of Preparation 2C.

C. 1,3,6'-Tri-N-Benzoyl-2'-N-(2,2,2-trichloroethoxycarbonyl)-Sisomicin

Prepare a solution of 2'-N-(2,2,2-trichloroethoxycarbonyl)-6'-N-benzoylsisomicin product of Preparation 2B in methanol (120 ml), and chloroform (60 ml), then with stirring add anhydrous sodium carbonate (20 g) followed by a dropwise addition of benzoic anhydride (12.1 g) in chloroform (40 ml). Stir the reaction mixture at room temperature for one hour, add chloroform (300 ml) and wash the organic solution with water (150 ml). Evaporate the organic phase in vacuo to a volume of about

100 ml, then to the resulting suspension add ether (500 ml) with agitation. Separate the resultant precipitate by filtration and wash with ether to obtain 1,3,6'-tri-N-benzoyl-2'-N-(2,2,2-trichloroethoxycarbonyl) sisomicin, which is used without further purification in the procedure of Preparation 2D.

D. 1,3,6'-Tri-N-Benzoylsisomicin

To a solution of 1,3,6'-tri-N-benzoyl-2'-N-(2,2,2-trichloroethoxycarbonyl)sisomicin product of Preparation 2C in methanol (250 ml) and acetic acid (30 ml), add with stirring zinc dust (30 g) and stir for 30 minutes at room temperature and at reflux temperature for 1 1/2 hours. Decant the reaction solution (from any undissolved zinc which may remain) into 2 N ammonium hydroxide (1.2 liters) and extract the aqueous mixture with chloroform (three 500 ml portions). Dry the combined extracts over potassium carbonate, filter and evaporate *in vacuo*. Dissolve the resultant residue in hot methanol (50 ml), add warm ethyl acetate (250 ml) and stir the mixture gently for 18 hours at room temperature. Separate the resultant precipitate by filtration, wash with ethyl acetate and dry *in vacuo* at 60°C to obtain 1,3,6'-tri-N-benzoylsisomicin; yield 7.0 g; m.p. 233 - 236°C; $[\alpha]_D^{26}$ + 97.4° (aqueous tetrahydrofuran, c=0.50).

E. 1,3,6'-Tri-N-Benzoyl-3"-N-Acetylsisomicin

To a solution of 1,3,6'-tri-N-benzoylsisomicin (16.o g) in tetrahydrofuran (300 ml) and water (200 ml) add with stirring over a 15-minute interval a solution of N-(2,2,2-trichloroethoxycarbonyloxy)succinimide (6.1 g) in tetrahydrofuran (70 ml). Stir the reaction mixture for 15 minutes, then add acetic anhydride (20 ml) and continue stirring the reaction mixture for one hour at room temperature. Add 500 ml of chloroform, then with

stirring add sodium carbonate (30 g). Separate the organic and aqueous phases, evaporate the organic phase *in vacuo*, dissolve the resultant residue in methanol (165 ml) and acetic acid (15 ml). Add zinc dust (20 g), stir for 30 minutes, then add additional acetic acid (5 ml). Stir the reaction mixture at reflux temperature for 1 hour, then cool and decant from any excess zinc into a mixture of ice (400 g) and concentrated ammonium hydroxide (200 ml). Extract the mixture with chloroform (three portions of 300 ml). Dry the combined extracts over potassium carbonate and evaporate *in vacuo*. Chromatograph the resultant residue on silica gel (about 300 g) eluting with a solvent mixture comprising chloroform: methanol:concentrated ammonium hydroxide (10:1:0.1). Evaporate the combined, like fractions containing the desired product as determined by thin layer chromatography, dissolve the resultant residue in chloroform (50 ml) and methanol (5 ml) and add to a stirred mixture of ether (700 ml) and hexane (350 ml). Collect the resultant precipitate by filtration, wash with ether, and dry *in vacuo* at 60°C to obtain 1,3,6'-tri-*N*-benzoyl-3"-*N*-acetylsisomicin; yield 9.3 g; m.p. ca. 220°C (decomp.); $[\alpha]_D^{26}$ + 121° (dimethylformamide, c=0.40).

## PREPARATION 3

## 1,3,6',3"-TETRA-N-ETHOXYCARBONYLGENTAMICIN $C_1$

A. 2'-*N*-benzyloxycarbonylgentamicin $C_1$

To a stirred solution of gentamicin $C_1$ (30 g) in dimethylsulfoxide (5.4 liters) add cupric acetate monohydrate (37.2 g). Stir until solution occurs, then add *N*-benzyloxycarbonyloxysuccinimide (22.2 g) in tetrahydrofuran (100 ml). Stir the reaction mixture for 30 minutes, pour into water (3 liters) extract continuously with chloroform and discard the chloroform extracts. To the

aqueous solution add acetyl acetone (20 ml), stir vigorously at room temperature for 3 hours, then filter. Pass the filtrate through IRA-401S ($OH^-$) resin (300 mg), then freeze-dry and chromatograph the resultant residue over silica gel (500 g) eluting with a solvent mixture comprising chloroform:methanol:15 % ammonium hydroxide (2:1:1). Evaporate the combined, like eluates containing the desired product as determined by thin layer chromatography to a residue of 2'-N-benzyloxycarbonylgentamicin $C_1$; yield 7.3 g.

B. 1,3,6',3"-Tetra-N-Ethoxycarbonyl-2'-N-Benzyloxycarbonylgentamicin $C_1$

To a solution of 2'-N-benzyloxycarbonylgentamicin $C_1$ (5.7 g) and sodium carbonate (19.6 g) in methanol (200 ml) and water (200 ml) at 0 - 4°C, add with stirring ethoxycarbonyl chloride (6.72 ml) and vigorously stir the reaction mixture for 24 hours at 0 - 4°C. Separate the resultant precipitate by filtration, wash with water (two portions of 100 ml), dry *in vacuo* at 45°C to a residue comprising 1,3,6',3"-tetra-N-ethoxycarbonyl-2'-benzyloxycarbonylgentamicin $C_1$. Additional product is obtained by extracting the filtrate with chloroform, drying the chloroform extracts over magnesium sulfate and evaporating the combined extracts *in vacuo*. Combine both the foregoing residues and purify by passing through silica gel (36 g) eluting with chloroform:methanol (98:2). Evaporate the combined eluates to obtain 1,3,6',3"-tetra-N-ethoxycarbonyl-2'-N-benzyloxycarbonylgentamicin $C_1$; yield 6.89 g.

C. 1,3,6',3"-Tetra-N-Ethoxycarbonylgentamicin $C_1$

Dissolve the 1,3,6',3"-tetra-N-ethoxycarbonyl-2'-N-benzyloxycarbonylgentamicin $C_1$ (6.89 g) product of Preparation 3 B in ethanol (200 ml) and hydrogenate utilizing 30 % palladium-on-charcoal catalyst (30 %, 0.5 g). Filter the reaction mixture, evaporate the filtrate and chromatograph the resultant residue over silica gel (25 g) eluting with chloroform:methanol (95:5). Evaporate the combined, like eluates to a residue comprising 1,3,6',3"-tetra-N-ethoxycarbonylgentamicin $C_1$, yield 5.1 g.

PREPARATION 4

1,3,6',3"-TETRA-N-ETHOXYCARBONYLGENTAMICIN $C_{2b}$

Subject gentamicin $C_{2b}$ to the series of reactions described in Preparations 3A, 3B and 3C to obtain 1,3,6',3"-tetra-N-ethoxycarbonylgentamicin $C_{2b}$.

PREPARATION 5

1,3,6'-TRI-N-BENZOYL-3"-N-ACETYL-4'(5')-DEHYDRO AMINOGLYCOSIDES

Subject each of the following aminoglycosides to the series of reactions described in Preparation 2(A)-(E): Antibiotic G-52, Antibiotic 66-40D, Antibiotic 66-40B, verdamicin, Antibiotic Mu-1, 5-deoxysisomicin, Antibiotic Mu-4, 5-episisomicin, 5-epi-azido-5-deoxysisomicin, and 1-N-ethylsisomicin. Isolate and purify each of the resultant products in a manner similar to that described in Preparation 2E to obtain the 1,3,6'-tri-N-benzoyl-3"-N-acetyl derivative of each of the aforenamed starting aminoglycosides.

PREPARATION 6

1,3,6',3"-TETRA-N-BENZYLOXYCARBONYL DERIVATIVES OF TOBRAMYCIN, GENTAMICIN $C_2$, AND GENTAMICIN $C_{2a}$

A. 1,3,6',3"-Tetra-N-benzyloxycarbonyltobramycin, Gentamicin $C_2$, and Gentamicin $C_{2a}$

Subject each of tobramycin, gentamicin $C_2$, gentamicin $C_{2a}$ and 3',4'-dideoxykanamycin B to the series of reac-

tions described in Preparations 1A, 1B, 1C and isolate each of the resultant products in a manner similar to that described to obtain, respectively, 1,3,6',3"-tetra-N-benzyloxycarbonyltobramycin, 1,3,6',3"-tetra-N-benzyloxycarbonylgentamicin $C_2$, 1,3,6',3"-tetra-N-benzyloxycarbonylgentamicin $C_{2a}$ and 1,3,6',3"-tetra-N-benzyloxycarbonyl-3',4'-dideoxykanamycin B.

## PREPARATION 7

## 1,3,6',3"-TETRA-N-BENZYLOXYCARBONYL DERIVATIVES OF ANTIBIOTICS JI-20A AND JI-20B

A. 6'-N-Benzyloxycarbonyl-Antibiotic JI-20A

To a solution of Antibiotic JI-20A (1.44 g) in water (1.5 ml), dimethylsulfoxide (58.5 ml) and triethylamine (0.6 ml) add cupric chloride (0.51 g). Stir for 15 minutes at room temperature, then add N-benzyloxycarbonyloxyphthalimide (0.910 g) and dissolve in dimethylsulfoxide (5 ml). After 1 hour add additional N-benzyloxycarbonyloxyphthalimide (0.6 g) in dimethylsulfoxide (4 ml) and stir. Pour the reaction mixture into ether, decant, add ether and stir, then decant again and repeat this procedure several times; Dissolve the resulting syrupy residue in 200 ml of methanol, bubble hydrogen sulfide through the methanol solution, separate the resulting copper sulfide salt precipitate by filtration, stir the filtrate with IRA-401S ($OH^{\ominus}$) resin (50 ml) and separate the resin by filtration. Evaporate the filtrate and chromatograph the resultant residue on silica gel (100 g) eluting with chloroform:methanol:28 % ammonium hydroxide (2:1:0.35). Combine the like fractions containing the desired product as determined by thin layer chromatography and evaporate to a residue comprising 6'-N-benzyloxycarbonyl-Antibiotic JI-20A.

B. 2'-N-Tert.-Butoxycarbonyl-6'-N-Benzyloxycarbonyl-Antibiotic JI-20A

Dissolve 6'-N-benzyloxycarbonyl-Antibiotic JI-20A (0.517 g) in methanol (15 ml), then add nickel (II) acetate tetrahydrate (0.21 g) and stir at room temperature for 15 minutes. Add N-tert.-butoxycarbonyloxyphthalimide (0.22 g) and stir for 3 hours while adding every hour additional portions of N-tert.-butoxycarbonyloxyphthalimide (0.2 g each). Bubble hydrogen sulfide through the reaction mixture, separate the resultant nickel salts by filtration, stir the filtrate with IRA-401S ($OH^{\ominus}$) resin (20 ml) and separate the resin by filtration. Evaporate the filtrate and chromatograph the resultant syrupy residue on 50 g of silica gel eluting with chloroform: methanol:28 % ammonium hydroxide (30:10:1). Combine the like fractions containing the desired product as determined by thin layer chromatography and evaporate to a residue comprising 2'-N-tert.-butoxycarbonyl-6'-N-benzyloxycarbonyl-Antibiotic JI-20A.

C. 1,3,6',3"-Tetra-N-Benzyloxycarbonyl-2'-N-Tert.-Butoxycarbonyl-Antibiotic JI-20A

Dissolve 2'-N-tert.-butoxycarbonyl-6'-N-benzyloxycarbonyl-AntibioticJI-20A (0.716 g) in methanol (15 ml) and stir (5 ml). Cool the solution to $0^{O}C$, add sodium carbonate (0.53 g) and then add benzyloxycarbonyl chloride (0.74 ml). Allow the reaction mixture to stand for 3 hours, then pour into 50 ml of water. Separate the resultant precipitate by filtration, wash the precipitate with water and then ether, then dry in vacuo to obtain 1,3,6',3"-tetra-N-benzyloxycarbonyl-2'-N-tert.-butoxycarbonyl-Antibiotic JI-20A.

D. Dissolve 1,3,6',3"-tetra-N-benzyloxycarbonyl-2'-N-tert.-butoxycarbonyl-Antibiotic JI-20A (1.121 g) in trifluoroacetic acid (5 ml). Allow the reaction mixture to stand at room temperature for 3 minutes, then add 50 ml of ether and separate the resultant precipitate by filtration. Dissolve the precipitate in methanol (30 ml) and stir with IRA-101S ($OH^{\ominus}$) ion exchange resin (10 ml). Separate the resin by filtration, add ether to the filtrate and separate the resultant precipitate by filtration to obtain 1,3,6',3"-tetra-N-benzyloxycarbonyl-Antibiotic JI-20A.

E. 1,3,6',3"-Tetra-N-Benzyloxycarbonyl-Antibiotic JI-20B

Treat Antibiotic JI-20B in a manner similar to that described in above Preparations 7A, 7B, 7C and 7D to obtain 1,3,6',3"-tetra-N-benzyloxycarbonyl-Antibiotic JI-20B.

## EXAMPLE 1

### 2'-HYDROXY-2'-DESAMINOGENTAMICIN $C_{1a}$ (3',4'-DIDEOXYGENTAMICIN B)

A. 1,3,6',3"-Tetra-N-Benzyloxycarbonyl-2'-Oximino-2'-Desamino-Gentamicin $C_{1a}$

Stir a solution of 1,3,6',3"-tetra-N-benzyloxycarbonyl-gentamicin $C_{1a}$ (7.0 g) in methanol (80 ml) with aqueous hydrogen perioxide (30 %, 5 g), then add a solution of sodium tungstate (0.3 g) in water (2 ml). Stir the reaction mixture at 15-20$^{o}$C keeping the solution at a pH of about 10-10.5 by periodic small additions of 1 N sodium hydroxide. Monitor the progress of the reaction via thin layer chromatography for almost complete consumption of the 2'-amino function (4-5 hours). Add the reaction mixture to water (700 ml) containing concentrated hydrochloric acid (2 ml). Extract the aqueous mixture with chloroform (two portions of 250 ml), wash the combined extracts with water, dry over magnesium sulfate and evaporate. Purify the resultant residue via rapid

chromatography on silica gel (200 g) eluting with 4 % chloroform in methanol. Combine the like eluates containing the mixture of syn and anti of the desired 2'-oximino product and evaporate in vacuo at 60°C to obtain an isomeric mixture of syn and anti 1,3,6',3"-tetray-N-benzyloxycarbonyl-2'-oximino-2'-desaminogentamicin $C_{1a}$; yield 5.70 g; 80 % theory, which is used without further purification in the procedure of following Example 1B.

B. 1,3,6',3"-Tetra-N-Benzyloxycarbonyl-2'-Oxo-2'-Desamino-Gentamicin $C_{1a}$

Stir at reflux temperature a solution of 1,3,6',3"-tetra-N-benzyloxycarbonyl-2'-oximino-2'-desaminogentamicin $C_{1a}$ (isomeric mixture prepared as described in Example 1A) and sodium bisulfite (5.5 g) in ethanol (65 ml) and water (28 ml). After stirring for 10 minutes, add additional sodium bisulfite (2.5 g) and continue stirring at reflux temperature for 45 minutes. Add acetic acid (15 ml), stir the reaction mixture at reflux temperature an additional 45 minutes, then pour into water (500 ml). Extract the aqueous mixture with chloroform, wash the combined chloroform extracts with aqueous sodium bicarbonate solution, dry the chloroform over sodium sulfate and evaporate to a residue comprising 1,3,6',3"-tetra-N-benzyloxycarbonyl-2'-oxo-2'-desaminogentamicin $C_{1a}$ which is used without further purification in the procedure of following Example 1C.

C. 1,3,6',3"-Tetra-N-Benzyloxycarbonyl-2'-Hydroxy-2'-Desamino-Gentamicin $C_{1a}$

Dissolve the 1,3,6',3"-tetra-N-benzyloxycarbonyl-2'-oxo-2'-desaminogentamicin $C_{1a}$ prepared in Example 1B in tetrahydrofuran (35 ml) and ethanol (35 ml), and with

stirring add a solution of sodium borohydride (1 g) in water (5 ml). Stir the reaction mixture at room temperature for 10 minutes, then carefully add acetic acid until the solution is at a pH of about 10. Pour the reaction mixture into a large volume of water and extract with chloroform. Wash the combined chloroform extracts with water, dry over magnesium sulfate and evaporate in vacuo and dry the resultant residue at 60°C in vacuo to obtain 1,3,6',3"-tetra-N-benzyloxycarbonyl-2'-hydroxy-2'-desaminogentamicin $C_{1a}$ which is used without further purification in the procedure of following Example 1D.

D. Dissolve the 1,3,6',3"-tetra-N-benzyloxycarbonyl-2'-hydroxy-2'-desaminogentamicin $C_{1a}$ prepared in Example 1C in dry tetrahydrofuran (12 ml) and add this solution dropwise to a stirred solution of sodium (1.5 g) in liquid ammonia (70 ml). Stir the reaction mixture for 10 minutes, then add methanol (20 ml) and water (100 ml) and evaporate in vacuo to a volume of about 75 ml. Pour onto excess IRC-50 ($H^{\oplus}$) resin, wash the resin with water, then elute with 1 N ammonium hydroxide. Evaporate the combined, like eluates containing the desired product as determined by thin layer chromatography, evaporate, and chromatograph the resultant residue on silica gel (50 g) eluting with solvent mixture comprising chloroform: methanol:concentrated ammonium hydroxide (2:1:0.2). Again evaporate the combined eluates containing the desired product as determined by thin layer chromatography, dissolve the resultant residue in water and pass through IRA-401 ($OH^{\ominus}$) resin. Lyophilize the combined eluates to a residue of 2'-hydroxy-2'-desaminogentamicin $C_{1a}$; yield 971 mg, 67 % theory; m.p. 110 - 114°C; $[\alpha]_D^{26}$ + 162.4° (water, c=0.33). Combustion analysis: Found: C, 49.2; H, 8.5; N, 11.8. $C_{19}H_{38}N_4O_8 \cdot H_2O$ req. C, 49.7; H, 8.6;

N, 12.0 %. pmr spectrum in deuterium oxide: 1.16 (s, 3, 4"-$CCH_3$), 2.47 (s, 3, 3"-$NCH_3$), 3.75 (dd, $J_{1"2"}$=4 Hz, $J_{2"3"}$=10.5 Hz, 1, $H_{2"}$), 4.00 (d, $J_{5"ax5"eq}$=12.5 Hz, 1, $H_{5"eq}$). 5.04 (d, $H_{1"2"}$=4 Hz, 1, $H_{1"}$) and 5.18 (d, $H_{1'2'}$= 3.5 Hz, 1, $H_{1'}$).

## EXAMPLE 2

## 1-N-(AMINOHYDROXYALKALNOYL)-2'-HYDROXY-2'-DESAMINO-GENTAMICIN $C_{1a}$

A. 3',6'-Di-N-Benzyloxycarbonyl-2'-Hydroxy-2'-Desamino-Gentamicin $C_{1a}$

Stir a solution of 2'-hydroxy-2'-desaminogentamicin $C_{1a}$ (710 mg) in dimethylsulfoxide (28 ml) at room temperature, then add powdered cupric acetate (600 mg) and nickelous acetate (750 mg). Continue stirring for 30 minutes, then add dropwise a solution of N-(benzyloxycarbonyloxy) phthalimide (1.05 g) in dry dimethylsulfoxide (6 ml) and continue stirring for 15 minutes. Add the reaction solution to aqueous ammonium hydroxide (2 N; 250 ml) and extract with chloroform (three 150 ml portions). Wash the combined organic extracts with water (50 ml), dry over potassium carbonate, filter and evaporate to a residue comprising 3,6'-di-N-benzyloxycarbonyl-2'-hydroxy-2'-desaminogentamicin $C_{1a}$, which is used without further purification in aqueous methanol in the procedure of Example 2B(1).

B. 3,6'-Di-N-Benzyloxycarbonyl-1-N-[S-γ-(benzyloxycarbonyl-amino)-α-Hydroxybutyryl]-2'-hydroxy-2'-desaminogentamicin $C_{1a}$

(1) The requisite active ester reagent N-[S-γ-(benzyloxycarbonylamino)-α-hydroxybutyryloxy]succinimide in tetrahydrofuran is prepared as follows. To a stirred solution of S-γ-(benzyloxycarbonylamino)-α-hydroxybutyric acid (0.60 g) and dry N-hydroxysuccinimide (0.38 g) in dichloromethane (10 ml) and ethyl acetate (10 ml) add dropwise a solution of N,N-dicyclohexylcarbodiimide (0.50 g) in dichloromethane (5 ml). Stir the mixture overnight with the exclusion of moisture. Filter the reaction mixture and wash the insoluble with ethyl acetate. Evaporate the combined filtrate and ethyl acetate washings in vacuo at 30°C. Dissolve the resultant residue comprising N-(S-γ-benzyloxycarbonylamino-α-hydroxybutyryloxy)succinimide in dry tetryhydrofuran (15 ml).

(2) To a stirred solution of 3',6'-di-N-benzyloxycarbonyl-2'-hydroxy-2'-desaminogentamicin $C_{1a}$ prepared in Example 2A in methanol (12 ml) and water (1.2 ml) add 10 ml of the tetrahydrofuran solution of N-[S-γ-(benzyloxycarbonylamino)-α-hydroxybutyryloxy]succinimide prepared in Example 2B(1) and stir the reaction mixture for 30 minutes, then add ammonium hydroxide (2 N; 10 ml) and evaporate the reaction mixture in vacuo to romove methanol and tetrahydrofuran. Extract the resultant residue with chloroform, dry the combined extracts over potassium carbonate, filter and evaporate. Chromatograph the resultant residue on silica gel (70 g) eluting with solvent mixture comprising chloroform:methanol:concentrated ammonium hydroxide (12:1:0.1). Combine the like eluates containing the desired product as determined by thin layer chromatography, evaporate and dry the resultant residue in vacuo at 60°C to obtain 3,6'-di-N-benzyloxycarbonyl-1-N-[S-γ-(benzyloxycarbonylamino)-α-hydroxybutyryl]-2'-hydroxy-2'-desaminogentamicin $C_{1a}$ as a white solid, yield 450 mg; 32 % theory; m.p. 200 - 202°C; $[\alpha]_D^{26}$ + 51.1° (tetrahydrofuran,

c=0.43). Combustion Analysis: Found: C, 57.2; H, 6.55; N, 6.9. Theory: $C_{47}H_{63}N_5O_{16}.2H_2O$ req C, 57.0; H, 6.8; N, 7.1 %.

C. 1-N-(S-γ-Amino-α-Hydroxybutyryl)-2'-Hydroxy-2'-Desamino-Gentamicin $C_{1a}$ (1-N-(S-γ-amino-α-hydroxybutyryl)-3',4'-Dideoxygentamicin B)

To 3,6'-di-N-benzyloxycarbonyl-1-N-[S-γ-(benzyloxycarbonylamino)-α-hydroxybutyryl]-2'-hydroxy-2'-desaminogentamicin $C_{1a}$ (400 mg) in tetrahydrofuran (30 ml) and water (4 ml) add acetic acid (0.25 ml) and 5 % palladium-on-carbon (500 mg) and shake the reaction mixture at one atmosphere of hydrogen for 24 hours. Dilute the reaction mixture with water, separate the catalyst by filtration and wash with 2 N ammonium hydroxide. Evaporate the combined filtrate and washings *in vacuo*, dissolve the resultant residue in water and pass down a column of IRA-401S ($OH^{\ominus}$) resin eluting slowly with water. Collect the eluates under nitrogen, combine the like eluates containing the desired product as determined by thin layer chromatography and lyophilize the combined eluates to obtain 1-N-(S-γ-amino-α-hydroxybutyryl)-2'-hydroxy-2'-desaminogentamicin $C_{1a}$ as a white amorphous solid, yield 156 mg, 70 % theory; m.p. 70 - 80°C; $[\alpha]_D^{26}$ + 109.5° (water, c=0.25). pmr. in $D_2O$ 1.17 (s, 3,4"-$CCH_3$), 2.47 (s, 3,3"-$NCH_3$), 4.08 (d, $J_{5"ax5"eq}$=13 Hz, 1, $H_{5"eq}$), 4.18 (dd, 1, $H_{2'''}$), 5.09 (d, $J_{H1"2"}$=4 Hz, 1, $H_{1"}$) and 5.29 (dd, $H_{1'2'}$=4 Hz, 1, $H_{1'}$).

D. 1-N-(S-β-Amino-α-hydroxypropionyl)-2'-Hydroxy-2'-Desamino-Gentamicin $C_{1a}$ (1-N-(S-β-amino-α-Hydroxypropionyl)-3',4'-Dideoxygentamicin B)

Prepare a solution of N-[S-β-(benzyloxycarbonylamino)-α-hydroxypropionyloxy]succinimide in tetrahydrofuran from

S-β-(benzyloxycarbonylamino)-α-hydroxypropionic acid and dry N-hydroxysuccinimide in a manner similar to that described in Example 2B(1) and add dropwise to a solution of 3,6'-di-N-benzyloxycarbonyl-2'-hydroxy-2'-desaminogentamicin $C_{1a}$ in aqueous methanol and isolate and purify the resultant product in a manner similar to that described in Example 2B(2) to obtain 3,6'-di-N-benzyloxycarbonyl-1-N-[S-β-(benzyloxycarbonylamino)-α-hydroxypropionyl]-2'-hydroxy-2'-desaminogentamicin $C_{1a}$. Hydrogenate the foregoing 3',6'-di-N-benzyloxycarbonyl derivative in aqueous tetrahydrofuran in the presence of palladium-on-carbon in a manner similar to that described in Example 2C to obtain 1-N-(S-β-amino-α-hydroxypropionyl)-2'-hydroxy-2'-desaminogentamicin $C_{1a}$.

E. 1-N-(S-δ-Amino-α-hydroxyvaleryl)-2'-Hydroxy-2'-Desamino-Gentamicin $C_{1a}$ (1-N-(S-δ-amino-α-hydroxyvaleryl)-3',4'-Dideoxygentamicin B)

In a manner similar to that described in Example 2B(1), prepare a solution of N-[S-δ-(benzyloxycarbonylamino)-α-hydroxyvaleryl]succinimide in tetrahydrofuran from S-δ-benzyloxycarbonylamino-α-hydroxyvaleric acid and dry N-hydroxysuccinimide, then add this solution dropwise to a solution of 3',6'-di-N-benzyloxycarbonyl-2'-hydroxy-2'-desaminogentamicin $C_{1a}$ in aqueous methanol in a manner similar to that described in Example 2B(2). Isolate and purify the resultant product in a manner similar to that described to obtain 3,6'-di-N-benzyloxycarbonyl-1-N-[S-δ-(benzyloxycarbonylamino)-α-hydroxyvaleryl]-2'-hydroxy-2'-desaminogentamicin $C_{1a}$. Hydrogenate the foregoing 3',6'-di-N-benzyloxycarbonyl derivative in aqueous tetrahydrofuran in the presence of palladium-on-carbon in a manner similar to that described in Example 2C to obtain 1-N-(S-δ-amino-α-hydroxyvaleryl)-2'-hydroxy-2'-desaminogentamicin $C_{1a}$.

EXAMPLE 3

2'-HYDROXY-2'-DESAMINOSISOMICIN

A. 1,3,6'-Tri-N-Benzoyl-3"-N-Acetyl-2'-Oximino-2'-Desaminosisomicin

Stir a solution of 1,3,6'-tri-N-benzoyl-3"-N-acetylsisomicin (8.9 g) in methanol (130 ml) and water (10 ml) with aqueous hydrogen perioxide (50 %, 4 ml), then add a solution of sodium tungstate (0.25 g) in water (3 ml). Stir the reaction mixture at room temperature maintaining a pH of about 9 - 10 by periodic small additions of aqueous sodium hydroxide solution (10 % w/v). After 3 hours, add additional hydrogen peroxide (1 ml) and sodium tungstate (0.12 g) and stir the reaction mixture overnight at room temperature. Add acetic acid (2 ml) to the reaction mixture, then pour the mixture into water (1 liter). Decant the aqueous mixture from the solid residue, rinse the solid residue with water and retain the residue. Combine the aqueous mixture with the water washings and extract with a chloroform:isopropanol (4:1) solvent mixture (three portions of 300 ml). Dissolve the original solid residue in the combined organic solvent extracts, dry the solution over sodium carbonate, filter and evaporate. Chromatograph the resultant residue on silica gel (200 g) eluting with a solvent mixture comprising chloroform:methanol:ammonium hydroxide (10:1:0.1). Combine the like fractions containing the desired product as determined by thin layer chromatography and evaporate to a residue comprising 1,3,6'-tri-N-benzoyl-3"-N-acetyl-2'-oximino-2'-desaminosisomicin, yield 3.1 g, which is used without further purification in the procedure of following Example 3B.

B. 2'-Hydroxy-2'-Desaminosisomicin

To a stirred solution of 1,3,6'-tri-N-benzoyl-3"-N-acetyl-2'-oximino-2'-desaminosisomicin prepared in Example 3A (3.1 g) in ethanol (50 ml) and water (20 ml), add sodium bisulfite (5 g) and stir at reflux temperature for 1 hour. Add acetic acid (6 ml) and water (15 ml) and subject the solution to slow distillation for 15 minutes, then cool. To the cooled solution containing 1,3,6'-tri-N-benzoyl-3"-N-acetyl-2'-oxo-2'-desaminosisomicin, add sodium carbonate (7.5 g) in small portions with stirring followed by sodium borohydride (1.7 g) in small portions. Stir the reaction mixture overnight, then dilute with isopropanol (400 ml), filter, wash the insoluble salts with isopropanol, then evaporate the combined filtrate and washings *in vacuo* to a residue comprising 1,3,6'-tri-N-benzoyl-3"-N-acetyl-2'-hydroxy-2'-desaminosisomicin.

To the foregoing residue add a solution of sodium hydroxide (7 g) in water (50 ml) and heat at reflux temperature under an atmosphere of argon for 60 hours. Cool, add dilute sulfuric acid until the solution is at a pH of about 10 and pour onto excess IRC-50 ($H^{\oplus}$) resin. Wash the resin with water, then elute with an excess of 1 N ammonium hydroxide. Evaporate the combined eluates and chromatograph the resultant residue on silica gel (100 g) eluting with the lower phase of a chloroform:methanol: 15 % ammonium hydroxide (2:1:1) solvent mixture. Evaporate the combined, like fractions containing the desired product as determined by thin layer chromatography, dissolve the resultant residue in water and pass through a short column of IRA-401S ($OH^{\ominus}$) resin. Elute with water and lyophilize the eluates to obtain 2'-hydroxy-2'-desaminosisomicin as a white amorphous solid.

EXAMPLE 4

1-N-(AMINOHYDROXYALKANOYL)-2'-HYDROXY-2'-DESAMINOSISOMICIN

A. 3,6'-Di-N-Benzyloxycarbonyl-2'-Hydroxy-2'-Desaminosisomicin

In a manner similar to that described in Example 2A, treat 2'-hydroxy-2'-desaminosisomicin in dimethylsulfoxide with cupric acetate and nickelous acetate followed by treatment with N-(benzyloxycarbonyloxy)phthalimide, and then reaction with aqueous ammonium hydroxide. Isolate and purify the resultant product in a manner similar to that described to obtain 3,6'-di-N-benzyloxycarbonyl-2'-hydroxy-2'-desaminosisomicin.

B. 3,6'-Di-N-Benzyloxycarbonyl-1-N-(S-Benzyloxycarbonylamino-α-Hydroxyalkanoyl)-2'-Hydroxy-2'-Desaminosisomicin

In a manner similar to that described in Examples 2B(2), 2C and 2D, treat a solution of 3,6'-di-N-benzyloxycarbonyl-2'-hydroxy-2'-desaminosisomicin in aqueous methanol with each of N-[S-γ-(benzyloxycarbonylamino)-α-hydroxybutyryloxy]succinimide, N-[S-β-(benzyloxycarbonylamino)-α-hydroxypropionyloxy]succinimide, and N-[S-δ-(benzyloxycarbonylamino)-α-hydroxyvaleryloxy]succinimide. Isolate and purify each of the resultant products in a manner similar to that described in Example 3B(2) to obtain, respectively, 3,6'-di-N-benzyloxycarbonyl-1-N-[S-γ-(benzyloxycarbonylamino)-α-hydroxybutyryl]-2'-hydroxy-2'-desaminosisomicin, 3,6'-di-N-benzyloxycarbonyl-1-N-[S-β-(benzyloxycarbonylamino)-α-hydroxypropyl]-2'-hydroxy-2'-desaminosisomicin, and 3,6'-di-N-benzyloxycarbonyl-1-N-[S-δ-(benzyloxycarbonylamino)-α-hydroxyvaleryl]-2'-hydroxy-2'-desaminosisomicin.

C. 1-N-Aminohydroxyalkanoyl - 2'-Hydroxy-2'-Desaminosisomicin

In a manner similar to that described in Example 1D,

treat a solution of each of the compounds prepared in Example 4B in tetrahydrofuran with sodium in liquid ammonia, then isolate and purify each of the resultant products in a manner similar to that described in Example 1D to obtain, respectively, 1-N-[S-γ-amino-α-hydroxybutyryl]-2'-hydroxy-2'-desaminosisomicin, 1-N-[S-β-amino-α-hydroxypropyl]-2'-hydroxy-2'-desaminosisomicin, and 1-N-[S-δ-amino-α-hydroxyvaleryl]-2'-hydroxy-2'-desaminosisomicin.

## EXAMPLE 5

## 2'-HYDROXY-2'-DESAMINOGENTAMICIN $C_1$

A. 1,3,6',3"-Tetra-N-Ethoxycarbonyl-2'-Oximino-2'-Desaminogentamicin $C_1$

To a solution of 1,3,6',3"-tetra-N-ethoxycarbonylgentamicin $C_1$ (0.5 g, 0.652 mmol) in methanol (6 ml) add sodium tungstate dihydrate (21.5 mg) in water (1 ml), then add hydrogen peroxide (0.2 ml, 30 % solution). Stir, add additional methanol to complete solution, adjust the pH from 4.8 to 7.8 by the dropwise addition of 2 N sodium hydroxide, then add additional sodium tungstate dihydrate (60 mg) in water (1 ml) and adjust the pH to 10.5 by the dropwise addition of 2 N sodium hydroxide. Continue stirring at room temperature for 2 hours. Extract the reaction mixture with chloroform (three portions of 75 ml), dry over magnesium sulfate and evaporate *in vacuo* to a residue comprising 1,3,6',3"-tetra-N-ethoxycarbonyl-2'-oximinogentamicin $C_1$ (yield 0.3 g) which is used without further purification in the procedure of Example 5B.

B. 1,3,6',3"-Tetra-N-Ethoxycarbonyl-2'-Oxo-2'-Desaminogentamicin $C_1$

To a solution of 1,3,6',3"-tetra-N-ethoxycarbonyl-2'-

oximino-2'-desaminogentamicin $C_1$ (0.1 g) in acetonitrile (0.3 ml) add 1 N hydrochloric acid (0.28 ml) containing acetaldehyde (0.02 ml) followed by water ( 5 ml). Extract the reaction mixture with ethyl acetate (three portions of 15 ml), dry over magnesium sulfate and evaporate *in vacuo* to a residue comprising 1,3,6',3"-tetra-N-ethoxycarbonyl-2'-oxo-2'-desaminogentamicin $C_1$ (yield 80 mg) which is used without further purification in the procedure of Example 5C.

C. 1,3,6',3"-Tetra-N-Ethoxycarbonyl-2'-Hydroxy-2'-Desaminogentamicin $C_1$

To a solution of 1,3,6',3"-tetra-N-ethoxycarbonyl-2'-oxo-2'-desaminogentamicin $C_1$ (80 mg) in dioxane (2 ml) and water (2 ml) at $0^oC$ with stirring add dropwise a solution of sodium borohydride (50 mg) in aqueous dioxane (1:1, 2 ml). Add acetone (3 ml) followed by water (5 ml). Extract with ethyl acetate (three portions of 15 ml), dry over magnesium sulfate and evaporate *in vacuo* to a residue comprising 1,3,6',3"-tetra-N-ethoxycarbonyl-2'-hydroxy-2'-desaminogentamicin $C_1$, which is used without further purification in the procedure of following Example 5D.

D. 2'-Hydroxy-2'-Desaminogentamicin $C_1$

To a solution of 1,3,6',3"-tetra-N-ethoxycarbonyl-2'-hydroxy-2'-desaminogentamicin $C_1$ (1.21 g) in dimethylsulfoxide (16 ml) add a solution of potassium hydroxide (2 g) in water (3 ml) at $25^oC$. Stir at room temperature for 16 hours, then add 2 N sulfuric acid dropwise until the solution is at about pH 10.5. Filter, dilute the reaction solution with water (50 ml), then add IR-50 ($H^{\oplus}$) resin until the reaction mixture is at pH 5. Filter the resin, wash the resin with water (200 ml) discarding the water washing. Wash the resin with 2 N

ammonium hydroxide and evaporate the ammonium hydroxide solution *in vacuo*. Dissolve the resultant residue in 10 % aqueous sodium hydroxide (5 ml) and heat in a teflon bomb for 16 hours at 80°C, then for 16 hours at 120°C. Acidify the reaction mixture to a pH of about 11 with 2 N sulfuric acid, filter, then add IR-50 ($H^{\oplus}$) resin until the reaction mixture is at a pH of about 4.5. Filter, wash the resin with water, discarding the water wash, then wash the resin with 2 N ammonium hydroxide. Evaporate the ammonium hydroxide wash *in vacuo*, dissolve the resultant residue in 90 % hydrazine hydrate and heat at reflux temperature for 16 hours. Evaporate, chromatograph the resultant residue over silica gel eluting with a solvent mixture comprising chloroform:isopropanol:17 % ammonium hydroxide (2:1:1). Combine the like eluates containing the desired product as determined by thin layer chromatography and evaporate to a residue of 2'-hydroxy-2'-desaminogentamicin $C_1$; 479 $(M+1)^+$, 478 $(M.^+)$, 421, 348, 330, 320, 302, 350, 332, 322, 304, 160, 158. PMR (100 MHz, $D_2O$) $\delta$ 0.97 (3H, d, J=6.3 Hz, $\underline{CH_3}$-CH), 1.14 (3H, s, $\underline{CH_3}$-$C_{4"}$), 2.24 (3H, s, $\underline{CH_3}$-N), 2.43 (3H, s, $\underline{CH_3}$-N), 3.27 (1H, d, J=12.5 Hz, H-5"ax), 3.73 (1H, q, J=4, 10 Hz, H-2"), 3.99 (1H, d, J=12.5 Hz, 5"eq), 5.01 (1H, d, J=4, H-1"), 5.12 (1H, d, J=3.5, H-1') ppm.

## EXAMPLE 6

### 1-N-(AMINOHYDROXYALKANOYL)-2'-HYDROXY-2'-DESAMINOGENTAMICIN $C_1$

A. 3,6'-Di-N-Benzyloxycarbonyl-2'-Hydroxy-2'-Desaminogentamicin $C_1$

To a solution of 2'-hydroxy-2'-desaminogentamicin $C_1$ (0.79) in dimethylsulfoxide (31.6 ml), add cupric acetate dihydrate (490 mg) and nickelous acetate tetrahydrate (6.16 mg). Stir until solution is complete, then add N-(benzyloxycarbonyloxy)phthalimide (1.147 g). Follow the course of the reaction via thin layer chromatography, adding 2 additional 50 mg portions of N-(benzyloxycarbonyloxy)phthalimide at intervals. When one new major spot has appeared on the thin layer chromatogram, add 2 N ammonium hydroxide (250 ml), extract with methylene chloride (eight portions of 100 ml), wash the methylene chloride extracts with water, then dry over magnesium sulfate and evaporate in vacuo to a residue comprising 3,6'-di-N-benzyloxycarbonyl-2'-hydroxy-2'-desaminogentamicin $C_1$, which is used without further purification in the procedure of Example 6B.

B. 3,6'-Di-N-Benzyloxycarbonyl-1-N-[S-β-(benzyloxycarbonylamino)-α-Hydroxypropionyl]-2'-Hydroxy-2'-Desaminogentamicin $C_1$

To a solution of the 3,6'-di-N-benzyloxycarbonyl-2'-hydroxy-2'-desaminogentamicin $C_1$ prepared in Example 6A in methanol (20 ml) and water (2 ml), with stirring add N-(S-β-benzyloxycarbonylamino-α-propionyloxy)succinimide (1.5 equivalents in three equal portions). Extract with methylene chloride (three portions of 150 ml), dry over magnesium sulfate, and evaporate, chromatograph the resultant residue over silica gel (40 g) eluting with a solvent mixture comprising chloroform:methanol:ammonium hydroxide (2:1:1). Combine the like fractions containing the desired product as determined by thin layer chromatography and evaporate the combined eluates in vacuo to a residue comprising 3,6'-di-N-benzyloxycarbonyl-1-N-[S-β-(benzyloxycarbonylamino)-α-hydroxypropionyl]-2'-hydroxy-2'-desaminogentamicin $C_1$, yield 0.34 g. Combustion Analysis: Found: C, 58.93; H, 6.48; N, 7.08. Theory: $C_{48}H_{65}N_5O_{16}.H_2O$ C, 59.01; H, 6.81; N,7.17.

C. 1-N-(S-β-Amino-α-Hydroxypropionyl)-2'-Hydroxy-2'-Desaminogentamicin $C_1$

To a solution of 3,6'-di-N-benzyloxycarbonyl-1-N-[S-β-(benzyloxycarbonylamino)-α-hydroxypropionyl]-2'-hydroxy-2'-desaminogentamicin $C_1$ (0.3 g) in aqueous tetrahydrofuran (1:10) (15 ml), add acetic acid (0.15 ml) and hydrogenate in the presence of 5 % palladium-on-charcoal (200 mg) at 60 psc for 16 hours. Filter, evaporate the filtrate and chromatograph the resultant residue over silica gel (2 g) eluting with a solvent mixture comprising chloroform:methanol:ammonium hydroxide (3:4:2). Combine the like eluates containing the desired product as determined by thin layer chromatography and evaporate *in vacuo* to a residue comprising 1-N-(S-β-amino-α-hydroxypropionyl)-2'-hydroxy-2'-desaminogentamicin $C_1$. Further purify by dissolving in water, pouring the aqueous solution over IRA-401S ($OH^{\ominus}$) resin (0.6 ml) eluting slowly with water. Collect the eluates under nitrogen and freeze dry the combined eluates to obtain 1-N-(S-γ-amino-α-hydroxypropionyl)-2'-hydroxy-2'-desaminogentamicin $C_1$, yield 75.7 mg, $[\alpha]_D^{26}$ + 99.7° (water, c=0.32). Combustion Analysis: C, 46.73; H, 7.81; N, 10.74. Calculated $C_{24}H_{47}N_5O_{16}\cdot 1.5H_2CO_3$, C, 46.5; H, 7.65; N, 10.63; PMR (100 MHz, $D_2O$) δ 0.99 (3H, d, J=6.8 Hz, $CH_3$-CH), 1.13 (3H, s, $CH_3$-$C_{4''}$), 2.27 (3H, s, $CH_3$-N), 2.43 (3H, s, $CH_3$-N), 5.03 (1H, d, J=3.5, H-1"), 5.15 (1H, d, J=3.7 Hz, H-1') ppm.

D. In a manner similar to that described in Example 6B, treat 3,6'-di-N-benzyloxycarbonyl-2'-hydroxy-2'-desaminogentamicin $C_1$ with each of N-(S-γ-benzyloxycarbonylamino-α-hydroxybutyryloxy)succinimide and N-(S-δ-benzyloxycarbonylamino-α-hydroxyvaleryloxy)succinimide and isolate and purify each of the resultant products in the

described manner to obtain 3,6'-di-N-benzyloxycarbonyl-1-N-(S-γ-amino-α-hydroxybutyryl)-2'-hydroxy-2'-desaminogentamicin $C_1$ and 3,6'-di-N-benzyloxycarbonyl-1-N-(S-δ-amino-α-hydroxyvaleryl)-2'-hydroxy-2'-desaminogentamicin $C_1$, respectively. Hydrogenate each of the foregoing products in the presence of 5 % palladium-on-charcoal in a manner similar to that described in Example 6C and isolate and purify each of the resultant products to obtain, respectively, 1-N-(S-γ-amino-α-hydroxybutyryl)-2'-hydroxy-2'-desaminogentamicin $C_1$ and 1-N-(S-δ-amino-α-hydroxyvaleryl)-2'-hydroxy-2'-desaminogentamicin $C_1$.

EXAMPLE 7

OTHER 2'-HYDROXY-2'-DESAMINO AMINOGLYCOSIDES

A. Subject each of the 1,3,6',3"-tetra-N-benzyloxycarbonyl-aminoglycoside derivatives prepared in Preparations 6 and 7 to a series of reactions similar to that described in Examples 1A-1D to obtain, respectively, 2'-hydroxy-2'-desaminotobramycin, 2'-hydroxy-2'-desaminogentamicin $C_2$, 2'-hydroxy-2'-desaminogentamicin $C_{2a}$, 2'-hydroxy-2'-desamino-3',4'-dideoxykanamycin A (also named 3',4'-dideoxy-kanamycin A), 2'-hydroxy-2'-desamino-Antibiotic JI-20A (also named gentamicin B), and 2'-hydroxy-2'-desamino-Antibiotic JI-20B.

B. Subject 1,3,6',3"-tetra-N-ethoxycarbonylgentamicin $C_{2b}$ to a series of reactions similar to that described in Examples 5A - 5D and isolate and purify the resultant product in a manner similar to that in Example 5D to obtain 2'-hydroxy-2'-desaminogentamicin $C_{2b}$.

C. Subject each of the 1,3,6'-tri-N-benzoyl-3"-N-acetyl-aminoglycoside derivatives prepared in Preparation 5 to

a series of reactions similar to those described in Example 3, and isolate and pruify each of the resultant products in a manner similar to that described in Example 3B to obtain, respectively, 2'-hydroxy-2'-desamino-Antibiotic G-52, 2'-hydroxy-2'-desamino-Antibiotic 66-40D, 2'-hydroxy-2'-desamino-Antibiotic 66-40B, 2'-hydroxy-2'-desaminoverdamicin, 2'-hydroxy-2'-desamino-Antibiotic Mu-1, 2'-hydroxy-2'-desamino-5-deoxysisomicin, 2'-hydroxy-2'-desamino-Antibiotic Mu-4, 2'-hydroxy-2'-desamino-5-episisomicin, 2'-hydroxy-2'-desamino-5-epi-azido-5-deoxysisomicin, and 2'-hydroxy-2'-desamino-1-N-ethylsisomicin.

## EXAMPLE 8

## 1-N-(ω-AMINO-α-HYDROXYALKANOYL)-2'-HYDROXY-2'-DESAMINO AMINOGLYCOSIDE DERIVATIVES

A. 3,6'-Di-N-Benzyloxycarbonyl-2'-Hydroxy-2'-Desamino Aminoglycosides

In a manner similar to that described in Example 2A, treat each of the 2'-hydroxy-2'-desamino aminoglycosides prepared in Examples 7A, 7B and 7C with powdered cupric acetate and nickelous acetate followed by treatment with N-(benzyloxycarbonyloxy)phthalimide. Isolate and purify each of the resultant products in a manner similar to that described to obtain the corresponding 3',6'-di-N-benzyloxycarbonyl derivative.

B. 3,6'-Di-N-Benzyloxycarbonyl-1-N-[S-ω-(benzyloxycarbonylamino)-α-Hydroxyalkanoyl]-2'-Hydroxy-2'-Desamino Aminoglycosides

(1) 3,6'-Di-N-Benzyloxycarbonyl-1-N-[S-γ-(benzyloxycarbonylamino)-α-Hydroxybutyryl]-2'-Hydroxy-2'-Desamino Aminoglycosides

In a manner similar to that described in Example 2B(2), treat each of the 3,6'-di-N-benzyloxycarbonyl-

2'-hydroxy-2'-desamino aminoglycosides prepared in Example 8A with N-(S-γ-benzyloxycarbonylamino-α-hydroxybutyryloxy)succinimide, then isolate and purify each of the resultant products in a manner similar to that described to obtain the corresponding 1-N-[S-γ-(benzyloxycarbonylamino)-α-hydroxybutyryl] derivative.

(2) 3,6'-Di-N-Benzyloxycarbonyl-1-N-[S-β-(benzyloxycarbonylamino)-α-hydroxypropionyl]-2'-Hydroxy-2'-Desamino Aminoglycosides

In a manner similar to that described in Examples 2D and 6B, treat each of the 3,6'-di-N-benzyloxycarbonyl-2'-hydroxy-2'-desamino aminoglycosides prepared in Examples 7A, 7B and 7C with N-(S-β-benzyloxycarbonylamino-α-hydroxypropionyloxy)succinimide and isolate and purify each of the resultant products in a manner similar to that described to obtain 1-N-[S-β-(benzyloxycarbonylamino)-α-hydroxypropionyl] derivative.

(3) 3,6'-Di-N-Benzyloxycarbonyl-1-N-[S-δ-(benzyloxycarbonylamino)-α-Hydroxyvaleryl]-2'-Hydroxy-2'-Desamino Aminoglycosides

In a manner similar to that described in Example 2E, treat each of the 3,6'-di-N-benzyloxycarbonyl-2'-hydroxy-2'-desamino aminoglycoside derivatives prepared in Examples 7A, 7B and 7C with N-(S-δ-benzyloxycarbonylamino-α-hydroxyvaleryl)succinimide and isolate and purify each of the resultant products in a manner similar to that described to obtain, respectively, the 1-N-[S-δ-(benzyloxycarbonylamino)-α-hydroxyvaleryl] derivative.

C. 1-N-(S-ω-Amino-α-Hydroxyalkanoyl)-2'-Hydroxy-2'-Desamino Aminoglycosides

(1) In a manner similar to that described in Example 2C, hydrogenate each of the 3,6'-di-N-benzyloxycarbonyl-1-N-[S-ω-(benzyloxycarbonylamino)-α-hydroxyalkanoyl]-2'-hydroxy-2'-desamino aminoglycoside derivatives prepared in Example 8B(1), (2) and (3) which were derived from the products of Examples 7A and 7B, isolate and purify each of the resultant products in a manner similar to that in Example 2C to obtain, respectively, 1-N-(S-γ-amino-α-hydroxybutyryl)-2'-hydroxy-2'-desaminotobramycin, 1-N-(S-γ-amino-α-hydroxybutyryl)-2'-hydroxy-2'-desaminogentamicin $C_2$, 1-N-(S-γ-amino-α-hydroxybutyryl)-2'-hydroxy-2'-desaminogentamicin $C_{2a}$, 1-N-(S-γ-amino-α-hydroxybutyryl)-2'-hydroxy-2'-desamino-3',4'-dideoxykanamycin B, 1-N-(S-γ-amino-α-hydroxybutyryl)-2'-hydroxy-2'-desaminogentamicin $C_{2b}$; 1-N-(S-β-amino-α-hydroxypropionyl)-2'-hydroxy-2'-desaminotobramycin, 1-N-(S-β-amino-α-hydroxypropionyl)-2'-hydroxy-2'-desaminogentamicin $C_2$, 1-N-(S-β-amino-α-hydroxypropionyl)-2'-hydroxy-2'-desaminogentamicin $C_{2a}$, 1-N-(S-β-amino-α-hydroxypropionyl)-2'-hydroxy-2'-desamino-3',4'-dideoxykanamycin B, 1-N-(S-β-amino-α-hydroxypropionyl)-2'-hydroxy-2'-desaminogentamicin $C_{2b}$; 1-N-(S-δ-amino-α-hydroxyvaleryl)-2'-hydroxy-2'-desaminotobramycin, 1-N-(S-δ-amino-α-hydroxyvaleryl)-2'-hydroxy-2'-desaminogentamicin $C_2$, 1-N-(S-δ-amino-α-hydroxyvaleryl)-2'-hydroxy-2'-desaminogentamicin $C_{2a}$, 1-N-(S-δ-amino-α-hydroxyvaleryl)-2'-hydroxy-2'-desamino-3',4'-dideoxykanamycin B, 1-N-(S-δ-amino-α-hydroxyvaleryl)-2'-hydroxy-2'-desaminogentamicin $C_{2b}$.

(2) In a manner similar to that described in Example 1D, treat each of the 3,6'-di-N-benzyloxycarbonyl-1-N-[S-ω-(benzyloxycarbonylamino)-α-hydroxyalkanoyl]-2'-hydroxy-2'-desamino aminoglycosides prepared in Example 8B(1), (2), and (3) which were derived from the products of Example 7C with sodium in liquid ammonia. Isolate and purify each of the resultant products in a manner similar to that described to obtain, respectively, 1-N-(S-γ-amino-α-hydroxybutyryl)-2'-hydroxy-2'-desamino-Antibiotic G-52, 1-N-(S-γ-amino-α-hydroxybutyryl)-2'-hydroxy-2'-desamino-Antibiotic 66-40D, 1-N-(S-γ-amino-α-hydroxybutyryl)-2'-hydroxy-2'-desamino-Antibiotic 66-40B, 1-N-(S-γ-amino-α-hydroxybutyryl)-2'-hydroxy-2'-desaminoverdamicin, 1-N-(S-γ-amino-α-hydroxybutyryl)-2'-hydroxy-2'-desamino-Antibiotic Mu-1, 1-N-(S-γ-amino-α-hydroxybutyryl)-2'-hydroxy-2'-desamino-5-deoxysisomicin, 1-N-(S-γ-amino-α-hydroxybutyryl)-2'-hydroxy-2'-desamino-Antibiotic Mu-4, 1-N-(S-γ-amino-α-hydroxybutyryl)-2'-hydroxy-2'-desamino-5-episisomicin, 1-N-(S-γ-amino-α-hydroxybutyryl)-2'-hydroxy-2'-desamino-5-epi-azido-5-deoxysisomicin, and 1-N-(S-γ-amino-α-hydroxybutyryl)-2'-hydroxy-2'-desamino-1-N-ethylsisomicin; 1-N-(S-β-amino-α-hydroxypropionyl)-2'-hydroxy-2'-desamino-Antibiotic G-52, 1-N-(S-β-amino-α-hydroxypropionyl)-2'-hydroxy-2'-desamino-Antibiotic 66-40D, 1-N-(S-β-amino-α-hydroxypropionyl)-2'-hydroxy-2'-desamino-Antibiotic 66-40B, 1-N-(S-β-amino-α-hydroxypropionyl)-2'-hydroxy-2'-desaminoverdamicin, 1-N-(S-β-amino-α-hydroxypropionyl)-2'-hydroxy-2'-desamino-Antibiotic Mu-1, 1-N-(S-β-amino-α-hydroxypropionyl)-2'-hydroxy-2'-desamino-5-deoxysisomicin, 1-N-(S-β-amino-α-hydroxypropionyl)-2'-hydroxy-2'-desamino-Antibiotic Mu-4, 1-N-(S-β-amino-α-hydroxypropionyl)-2'-hydroxy-2'-desamino-5-episisomicin, 1-N-(S-β-amino-α-hydroxypropionyl)-2'-hydroxy-2'-desamino-5-epi-azido-5-deoxysisomicin, and 1-N-(S-β-amino-α-hydroxypropionyl)-2'-hydroxy-2'-desamino-1-N-ethylsiso-

micin; 1-N-(S-δ-amino-α-hydroxyvaleryl)-2'-hydroxy-2'-desamino-Antibiotic G-52, 1-N-(S-δ-amino-α-hydroxyvaleryl)-2'-hydroxy-2'-desamino-Antibiotic 66-40D, 1-N-(S-δ-amino-α-hydroxyvaleryl)-2'-hydroxy-2'-desamino-Antibiotic 66-40B, 1-N-(S-δ-amino-α-hydroxyvaleryl)-2'-hydroxy-2'-desaminoverdamicin, 1-N-(S-δ-amino-α-hydroxyvaleryl)-2'-hydroxy-2'-desamino-Antibiotic Mu-1, 1-N-(S-δ-amino-α-hydroxyvaleryl)-2'-hydroxy-2'-desamino-5-deoxysisomicin, 1-N-(S-δ-amino-α-hydroxyvaleryl)-2'-hydroxy-2'-desamino-Antibiotic Mu-4, 1-N-(S-δ-amino-α-hydroxyvaleryl)-2'-hydroxy-2'-desamino-5-episisomicin, 1-N-(S-δ-amino-α-hydroxyvaleryl)-2'-hydroxy-2'-desamino-5-epi-azido-5-deoxysisomicin, and 1-N-(S-δ-amino-α-hydroxyvaleryl)-2'-hydroxy-2'-desamino-1-N-ethylsisomicin.

## EXAMPLE 9

### ACID ADDITION SALTS

A. Sulfate Salts (Sulfuric Acid Addition Salts)

Dissolve 5.0 g of 2'-hydroxy-2'-desaminogentamicin $C_{1a}$ in 25 ml water and adjust the pH of the solution to 4.5 with 1 N sulfuric acid. Pour into about 300 ml of methanol with vigorous agitation, continue the agitation for about 10 - 20 minutes and filter. Wash the precipitate with methanol and dry at about 60°C *in vacuo* to obtain 2'-hydroxy-2'-desaminogentamicin $C_{1a}$ sulfate.

In like manner, the sulfate salt of the compounds of Examples 2 to 8 are prepared.

B. Hydrochloride Salts

Dissolve 5.0 g of 2'-hydroxy-2'-desaminogentamicin $C_{1a}$ in 25 ml of water. Acidify with 2 N hydrochloric acid to pH 5. Lyophilize to obtain 2'-hydroxy-2'-desaminogentamicin $C_{1a}$ hydrochloride.

In like manner, the hydrochloride salt of the compounds of Examples 2 to 8 are prepared.

The 2'-hydroxy-2'-desamino-4,6-di-O-(aminoglycosyl)-1,3-diaminocyclitols of this invention (particularly those wherein the 6-O-aminoglycosyl is 6-O-garosaminyl and wherein the 1,3-diaminocyclitol is 2-deoxystreptamine) and their non-toxic, pharmaceutically acceptable acid addition salts, in general, exhibit broad spectrum antibacterial activity and possess an improved antibacterial spectrum compared to that of the parent antibiotics. This improved spectrum consists of enhanced potency of the claimed compounds against organisms resistant to the parent compound. Thus, for example, compounds of this invention, e.g. 2'-hydroxy-2'-desamino-4-O-aminoglycosyl-6-O-garosaminyl-2-deoxystreptamines, are more active against organisms which inactivate the parent antibiotics by acetylation of the 2'-amino group. In addition to the foregoing, the 2'-hydroxy-2'-desamino-4,6-di-O-(aminoglycosyl)-1,3-diaminocyclitols and their non-toxic, pharmaceutically acceptable acid addition salts are, in general, less acutely toxic than their precursor 2'-amino parent antibiotics.

Particularly valuable are 2'-hydroxy-2'-desamino-gentamicin $C_1$ and 2'-hydroxy-2'-desaminogentamicin $C_{1a}$, which derivatives are broad spectrum antibacterial agents, being active against gram positive bacteria (e.g. Staphylococcus aureus) and gram negative bacteria (e.g. Escherichia coli and Pseudomonas aeruginosa) as determined by standard dilution tests, including bacteria resistant to the parent compound.

Most valuable compounds of this invention are the 1-N-(ω-amino-α-hydroxy) derivatives of 2'-hydroxy-2'-desaminogentamicin $C_{1a}$, particularly 1-N-(S-γ-amino-α-hydroxybutyryl)-2'-hydroxy-2'-desaminogentamicin $C_{1a}$ and 1-N-(S-β-amino-α-hydroxypropionyl)-2'-hydroxy-2'-desaminogentamicin $C_{1a}$ which exhibit broader spectra of antibacterial activity than the precursor 1-N-unsubstituted-2'-hydroxy-2'-desaminogentamicin $C_{1a}$ or the corresponding 1-N-(S-ω-amino-α-hydroxyalkanoyl) gentamicin $C_{1a}$. These preferred compounds possess improved potency versus bacteria resistant to the aforementioned precursor compounds, being active against 2"-adenylating and 3-acetylating strains of bacteria. Additionally, the foregoing 1-N-(ω-amino-α-hydroxy) derivatives of 2'-hydroxy-2'-desaminogentamicin $C_{1a}$ are advantageously less acutely toxic than the corresponding 1-N-(ω-amino-α-hydroxy)gentamicin $C_{1a}$.

The 2'-hydroxy-2'-desamino compounds of this invention can be used alone or in combination with other antibiotic agents to prevent the growth or reduce the number of bacteria in various environments. They may be used, for example, to disinfect laboratory glassware, dental and medical equipment contaminated with Staphylococcus aureus or other bacteria inhibited by the 2'-hydroxy-2'-desamino derivatives of this invention.

The activity of the 2'-hydroxy-2'-desamino-4,6-di-O-(aminoglycosyl)-1,3-diaminocyclitols against gram negative bacteria renders them useful for combatting infections caused by gram negative organisms, e.g. species of Proteus and Pseudomonas. The 2'-hydroxy-2'-desamino-4,6-di-O-(aminoglycosyl)-1,3-diaminocyclitols, e.g. 1-N-(S-γ-amino-α-hydroxybutyryl)-2'-hydroxy-2'-desaminogentamicin $C_{1a}$ and 1-N-(S-β-amino-α-hydroxypropionyl)-2'-hydroxy-2'-desaminogentamicin $C_{1a}$, have veterinary applications, particularly in the treatment of mastitis in cattle and Salmonella-induced diarrhea in domestic animals such as the dog and the cat.

The dosage administered of the 2'-hydroxy-2'-desamino-4,6-di-O-(aminoglycosyl)-1,3-diaminocyclitols will be dependent upon the age and wieght of the animal species being treated, the mode of administration, and the type and severity of bacterial infection being prevented or reduced. In general, the dosage employed to combat a given bacterial infection will be similar to the dosage requirements of the corresponding 4,6-di-O-(aminoglycosyl)-1,3-diaminocyclitol.

The compounds of this invention may be administered orally. They may also be applied topically in the form of ointments, both hydrophilic and hydrophobic, in the form of lotions which may be aqueous, non-aqueous or of the emulsion type or in the form of creams. Pharmaceutical carriers useful in the preparation of such formulations will include, for example, such substances as water, oils, greases, polyesters, polyols and the like.

For oral administration, the compounds of this invention may be compounded in the form of tablets, capsules, elixirs or the like or may even be admixed with animal feed. It is in these dosage forms that the antibacterials are most effective for treating bacterial infections of the gastrointestinal tract, which infections cause diarrhea.

In general, the topical preparations will contain from about 0.1 to about 3.0 g of a 2'-hydroxy-2'-desamino-4,6-di-O-(aminoglycosyl)-1,3-diaminocyclitol derivative per 100 g of ointment, creams or lotion. The topical preparations are usually applied gently to lesions from about 2 to about 5 times a day.

The antibacterials of this invention may be utilized in liquid form such as solutions, suspensions and the like for otic and optic use and may also be administered parenterally via intramuscular injection. The injectable solution or suspension will usually be administered at from about 1 mg to about

0000473

10 mg of antibacterial per kilogram of body weight per day divided into about 2 to about 4 doses. The precise dose depends on the stage and severity of the infection, the susceptibility of the infecting organism to the antibacterial and the individual characteristics of the animal species being treated.

The following formulations are to exemplify some of the dosage forms in which the antibacterial agents of this invention and their derivatives may be employed.

Formulation 1

| Tablet | 10 mg Tab. | 25 mg Tab. | 100 mg Tab. |
|---|---|---|---|
| 1-N-(S-γ-amino-α-hydroxybutyryl)-2'-hydroxy-2'-desamino-gentamicin $C_{1a}$ | 10.5* mg | 26.25* mg | 105.0* mg |
| Lactose, impalpable powder | 197.50 mg | 171.25 mg | 126.00 mg |
| Corn starch | 25.00 mg | 25.00 mg | 35.00 mg |
| Polyvinylpyrrolidone | 7.50 mg | 7.50 mg | 7.50 mg |
| Magnesium Stearate | 2.50 mg | 2.50 mg | 3.50 mg |

* 5 % excess

Procedure

Prepare a slurry consisting of the 1-N-(S-γ-amino-α-hydroxybutyryl)-2'-hydroxy-2'-desaminogentamicin $C_{1a}$, lactose and polyvinylpyrrolidone. Spray dry the slurry. Add the corn starch and magnesium stearate. Mix and compress into tablets.

Formulation 2

| Ointment | |
|---|---|
| 1-N-(S-γ-amino-α-hydroxybutyryl)-2'-hydroxy-2'-desaminogentamicin $C_{1a}$ | 1.9 g |
| Methylparaben, USP | 0.5 g |
| Polyparaben, USP | 0.1 g |
| Petrolatum | to 1000 g |

Procedure

(1) Melt the petrolatum.

(2) Mix the 1-N-(S-γ-amino-α-hydroxybutyryl)-2'-hydroxy-2'-desaminogentamicin $C_{1a}$, methylparaben and propylparaben with about 10 % of the molten petrolatum.

(3) Pass the mixture through a colloid mill.

(4) Add the remainder of the petrolatum with agitation and cool the mixture until it becomes semi-solid. At this stage the product may be put into suitable containers.

Ointments of other 2'-hydroxy-2'-desamino-4,6-di-O-(aminoglycosyl)-1,3-diaminocyclitols of this invention are prepared by substituting an equivalent quantity of 2'-hydroxy-2'-desamino-4,6-di-O-(aminoglycosyl)-1,3-diaminocyclitol or an acid addition salt thereof, for 1-N-(S-γ-amino-α-hydroxybutyryl)-2'-hydroxy-2'-desaminogentamicin $C_{1a}$ in the foregoing example and by following substantially the procedure of the example.

Formulation 3

| Injectable Solution | Per 2.0 ml Vial | Per 50 Liters |
|---|---|---|
| 1-N-(S-β-amino-α-hydroxypropionyl)-2'-hydroxy-2'-desaminogentamicin $C_{1a}$ | 84* mg | 2100* g |
| Methylparaben, USP | 3.6 mg | 90.0 g |
| Propylparaben, USP | 0.4 mg | 10.0 g |
| Sodium bisulfite, USP | 6.4 mg | 160.0 g |
| Disodium Ethylenediamine tetraacetate dihydrate, R.G. | 0.2 mg | 5.0 g |
| Water, USP q.s. | 2.0 ml | 50.0 liters |

*Includes a 5 % manufacturing overcharge

Procedure: For a 50.0 liter batch

Charge approximately 35 liters of water for injection to a suitable stainless steel jacketed vessel and heat to about

70°C. Charge the methylparaben and propylparaben to the heated water for injection and dissolve with agitation. When the parabens are completely dissolved, cool the contents of the tank to 25 - 30°C by circulating cold water through the tank jacket. Sparge the solution with nitrogen gas for at least 10 minutes and keep covered with nitrogen during subsequent processing. Charge and dissolve the disodium EDTA and sodium bisulfite. Charge and dissolve the 1-N-(S-β-amino-α-hydroxypropionyl)-2'-hydroxy-2'-desaminogentamicin $C_{1a}$ sulfate. Bring the batch volume up to 50.0 liters with water for injection and agitate until homogeneous.

Under sterile conditions, filter the solution through a suitable bacteria retentive filter collecting the filtrate in a filling tank.

Fill the filtrate aseptically into sterile pyrogen-free multiple dose vials, stopper and seal.

In like manner, injectable solutions of other 2'-hydroxy-2'-desamino-4,6-di-O-(aminoglycosyl)-1,3-diaminocyclitols and especially acid addition salts of such antibacterial agents, may be prepared by substituting an equivalent quantity of such compounds for 1-N-(S-β-amino-α-hydroxypropionyl)-2'-hydroxy-2'-desaminogentamicin $C_{1a}$ sulfate and by following the procedure set forth above.

CLAIMS

1. Process for the preparation of a 2'-hydroxy-2'-desamino-4,6-di-O-(aminoglycosyl)-1,3-diaminocyclitol having an amino function in position 6', characterized in that an appropriate 4,6-di-O-(aminoglycosyl)-1,3-diaminocyclitol having an amino function in position 2' wherein all other amino functions are protected by a protecting group, is oxidized by means of hydrogen peroxide in an inert solvent in the presence of tungstate ion at a pH of about 9 to about 11, whereby the molar ratio of hydrogen peroxide to starting compound is at least 2:1; that the so-obtained 2'-oximino-4,6-di-O-(aminoglycosyl)-1,3-diaminocyclitol is subjected to a cleavage of the oxime function; that the so-obtained 2'-oxo-4,6-di-O-(aminoglycosyl)-1,3-diaminocyclitol is reduced at the oxo group; and that the so-obtained compound is subjected to a removal of the protecting groups, followed, if desired and appropriate, by transformation into a 1-N-(ω-amino-α-hydroxyalkanoyl)-, 1-N-$CH_2X$-, 5-deoxy-, 5-epi-, 5-epi-azido-5-deoxy- and/or 5-epi-fluoro-5-deoxy-derivative, whereby X is as herein defined.

2. Process according to claim 1, characterized in that

a) the oxidation in the first step is carried out in an aqueous lower alkanol as a solvent;

b) the cleavage of the oxime function in the second step is carried out, preferably by means of sodium bisulfite followed by mild hydrolysis, or by treatment in an acidic hydrolytic medium in the presence of acetaldehyde;

c) the reduction in the third step is carried out by means of an alkali metal borohydride, preferably sodium borohydride; and

d) the amino protective groups are groups susceptible to reductive cleavage or to basic hydrolysis, preferably benzyloxycarbonyl, ethoxycarbonyl or methoxycarbonyl.

3. Process according to claim 1 or claim 2, characterized in that the starting compound used is Antibiotic JI-20A whereby the 2'-hydroxy-2'-desamino-Antibiotic JI-20A is obtained which is the same as gentamicin B.

4. 2'-Hydroxy-2'-desamino-4,6-di-O-(aminoglycosyl)-1,3-diaminocyclitols having amino functions in positions 6' and 3" and hydroxyl groups in the positions 2" and 4", whenever prepared by the process of claim 1 or claim 2.

5. A 2'-hydroxy-2'-desamino-4,6-di-O-(aminoglycosyl)-1,3-diaminocyclitol selected from the group consisting of

2'-hydroxy-2'-desaminogentamicin $C_1$,
2'-hydroxy-2'-desaminogentamicin $C_{1a}$,
2'-hydroxy-2'-desaminogentamicin $C_2$,
2'-hydroxy-2'-desaminogentamicin $C_{2a}$,
2'-hydroxy-2'-desaminogentamicin $C_{2b}$,
2'-hydroxy-2'-desaminosisomicin,
2'-hydroxy-2'-desaminoverdamicin,
2'-hydroxy-2'-desamino-Antibiotic G-52,
2'-hydroxy-2'-desamino-Antibiotic 66-40B,
2'-hydroxy-2'-desamino-Antibiotic 66-40D,
2'-hydroxy-2'-desamino-Antibiotic JI-20B,
2'-hydroxy-2'-desaminotobramycin; and
2'-hydroxy-2'-desamino-3',4'-dideoxykanamycin B;
the 5-deoxy, 5-epi-, 5-epi-azido-5-deoxy derivatives thereof;
2'-hydroxy-2'-desamino-Antibiotic Mu-1 and
2'-hydroxy-2'-desamino-Antibiotic Mu-4;

the 1-N-monosubstituted derivatives of the foregoing wherein the substituent R is either an (ω-amino-α-hydroxyalkanoyl) group having from 3 to 5 carbon atoms, or a group $CH_2X$ wherein X represents hydrogen, alkyl, cycloalkyl, alkenyl, cycloalkylalkyl, aryl, aralkyl, hydroxyalkyl, aminoalkyl, alkylaminoalkyl, aminohydroxyalkyl, and alkylaminohydroxyalkyl, said aliphatic radicals having up to 7 carbon atoms, and, when substituted by both hydroxy and amino having said substituents attached to different carbon atoms;
and the pharmaceutically acceptable acid addition salts thereof.

6. A compound according to claim 5, being a 1-N-(ω-amino-α-hydroxyalkanoyl) derivative of said 2'-hydroxy-2'-desamino-4,6-di-O-(aminoglycosyl)-1,3-diaminocyclitol.

7. A compound according to claim 6, being a 1-N-(ω-amino-α-hydroxyalkanoyl) derivative of a 2'-hydroxy-2'-desamino-4-O-garosaminyl-2-deoxystreptamine.

8. A compound according to claim 7, wherein the 1-N-substituent is a β-amino-α-hydroxypropionyl- or a γ-amino-α-hydroxybutyryl-group.

9. Compounds according to claim 8, being:

1-N-(S-β-amino-α-hydroxypropionyl)-2'-hydroxy-2'-desamino-gentamicin $C_1$;
1-N-(S-β-amino-α-hydroxypropionyl)-2'-hydroxy-2'-desamino-gentamicin $C_{1a}$;
1-N-(S-γ-amino-α-hydroxybutyryl)-2'-hydroxy-2'-desamino-gentamicin $C_1$; and
1-N-(S-γ-amino-α-hydroxybutyryl)-2'-hydroxy-2'-desamino-gentamicin $C_{1a}$;
and acid addition salts thereof.

10. A pharmaceutical composition comprising as an active ingredient a compound as defined in any one of claims 4 to 7.

European Patent Office

# EUROPEAN SEARCH REPORT

0000473

Application number

EP 78 10 0207

## DOCUMENTS CONSIDERED TO BE RELEVANT

| Category | Citation of document with indication, where appropriate, of relevant passages | Relevant to claim |
|---|---|---|
| X | REAGENTS FOR ORGANIC SYNTHESIS; Fieser and Fieser, vol. 1, Wiley 1967 New York.<br>* Page 475 *<br>-- | 1,2 |
| X | STICKSTOFFVERBINDUNGEN I, Band 10, Teil 4, Houben - Weyl G.Thieme Verlag, Stuttgart 1968.<br>* Page 269 *<br>-- | 1,2 |
| X | FR - A - 2 201 872 (SCHERICO)<br>* Page 85, line 19 - page 87, line 4 *<br>-- | 5,10 |
| X | FR - A - 2 289 202 (PFIZER)<br>* Pages 12-13 *<br>-- | 5 |
| X | THE JOURNAL OF ANTIBIOTICS 29(4) (1976) pages 319-353.<br>* Page 320: Gentamicin $A_3$, Gentamicin B, Kanamycin A *<br>-- | 5,10 |
| | THE JOURNAL OF ANTIBIOTICS 27 (11) (1974) pages 851-858<br>* Pages 854-856 *<br>-- | 5,6,7,8 |
| | FR - A - 2 293 941 (BRISTOL-MYERS)<br>* Page 35 *<br>-- | 5,6 |
| | FR - A - 2 218 879 (BRISTOL-MYERS)<br>* Page 34, line 32; page 35 *<br>---- | 5,6 |

CLASSIFICATION OF THE APPLICATION (Int. Cl.²)

C 07 H 15/22
A 61 K 31/70

TECHNICAL FIELDS SEARCHED (Int.Cl.²)

C 07 H 15/22
A 61 K 31/70

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 13-09-1978 | VERHULST |